(19) Europäisches Patentamt
European Patent Office
Office européen des brevets

(11) **EP 3 348 256 B1**

(12) **EUROPEAN PATENT SPECIFICATION**

(45) Date of publication and mention
of the grant of the patent:
**21.10.2020 Bulletin 2020/43**

(21) Application number: **16844378.6**

(22) Date of filing: **07.09.2016**

(51) Int Cl.:
*A61K 9/00* *(2006.01)*   *A61M 37/00* *(2006.01)*
*A61K 38/00* *(2006.01)*   *A61K 39/00* *(2006.01)*
*A61K 39/39* *(2006.01)*   *A61K 47/26* *(2006.01)*
*A61K 47/36* *(2006.01)*

(86) International application number:
**PCT/JP2016/076271**

(87) International publication number:
**WO 2017/043517 (16.03.2017 Gazette 2017/11)**

(54) **MICRONEEDLE ARRAY**

MIKRONADELARRAY

RÉSEAU DE MICRO-AIGUILLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.09.2015 JP 2015176346**

(43) Date of publication of application:
**18.07.2018 Bulletin 2018/29**

(73) Proprietor: **FUJIFILM Corporation
Tokyo 106-8620 (JP)**

(72) Inventors:
• **KABATA, Koki**
**Ashigarakami-gun**
**Kanagawa 258-8577 (JP)**
• **YOSHIDA, Junya**
**Ashigarakami-gun**
**Kanagawa 258-8577 (JP)**
• **OYAMADA, Takayoshi**
**Ashigarakami-gun**
**Kanagawa 258-8577 (JP)**

(74) Representative: **Hoffmann Eitle
Patent- und Rechtsanwälte PartmbB
Arabellastraße 30
81925 München (DE)**

(56) References cited:
**EP-A1- 2 815 784**       **WO-A1-2013/122160**
**WO-A1-2014/077242**     **JP-A- 2013 162 982**
**JP-A- 2013 162 982**

**Description**

Field of the Invention

The present invention relates to a microneedle array.

Description of the Related Art

As a method of administering a medicament to the surface of an organism such as the skin or the mucous membrane, a method of adhering a liquid substance or a powdery substance to the surface of an organism may be exemplified. Further, in biopharmaceuticals which have been attracting attention recently, since it is extremely difficult to enter a barrier layer through infiltration, a method of administering the pharmaceuticals through an injection is selected.

Further, a method of administering a medicament used to administer an appropriate amount of medicament and achieve sufficient drug efficacy, a method of injecting a medicament into the skin without pain by microneedles penetrating a horny barrier layer using a microneedle array in which microneedles (needle-like projections) which contain a medicament and have a high aspect ratio are formed has been attracting attention. For example, a self-dissolving microneedle array that uses a substance having solubility in vivo as a base material has been reported. In the self-dissolving microneedle array, a medicament can be intradermally administered by allowing the base material to hold a medicament and the base material being self-dissolved during insertion of microneedles into the skin.

Patent Document 1 describes a transdermal absorption sheet made of a polymer, including: a sheet portion; and a needle-like projection which contains a medicament and is formed on the surface of the sheet portion, in which the concentration of the medicament contained in the needle-like projection continuously decreases toward the root of the needle-like projection from the tip thereof; and a method of producing the same.

Patent Document 2 describes a microneedle which is a pyramidal, prismatic, conical, or columnar fine needle, in which the main component material of the fine needle is a mixture of maltose and dextran or trehalose and contains a functional substance acting on the skin, and the main component material is dissolved so that the functional substance is supplied to the skin by the fine needle being inserted into the skin.

Patent Document 3 describes a method of producing a microneedle sheet including: a step of filling a conical recess, which extends in a tapered shape by a first predetermined length from a first surface of a stamper toward a second surface facing the first surface, with a first microneedle raw material; and a step of forming a needle tip layer which extends only by a second predetermined length shorter than the first predetermined length by allowing the first microneedle raw material filling the recess to stand in an environment at a predetermined relative humidity and drying the first microneedle raw material, in which the predetermined relative humidity is in a range of 60% to 99%, and the solidification rate of the first microneedle raw material in the inner wall of the recess is suppressed.

Patent document 4 discloses a manufacturing method for a microneedle sheet, in which first a thin film of polymer solution containing drug is formed in a mold, followed by a polymer solution not containing drug to form the microneedles.

Prior Art Documents

Patent Documents

Patent Document 1 JP2011-224332A
Patent Document 2 JP2005-154321A
Patent Document 3 JP5587647B
Patent Document 4 JP2013-162982A

**SUMMARY OF THE INVENTION**

In a case where the method of adhering a medicament which is a liquid substance or a powdery substance to the surface of an organism is used, since the region to which the medicament adheres is limited to the surface of the skin, the adhering medicament is occasionally removed due to perspiration or contact with foreign matter. Therefore, it is difficult to administer an appropriate amount of medicament. Further, according to such a method of using infiltration resulting from diffusion of the medicament, since the infiltration of the medicament is inhibited by a horny barrier layer, it is difficult to obtain sufficient drug efficacy. In addition, administration of the medicament through an injection is associated with pain or infection risk because the injection needs to be performed by medical workers.

A method of injecting a medicament into the skin using a microneedle array has been attracting attention as a replacement of the method of administering a medicament through an injection, but this method using a microneedle array is difficult to obtain the same drug efficacy as the administration method through an injection. Particularly, in a case of a large animal assuming a human, it is difficult to obtain sufficient drug efficacy by stably administering a predetermined amount of medicament because the skin of the large animal is thicker than that of a small animal such as a mouse or a rat. In JP2011-224332A, JP2005-154321A, and JP5587647B, there is no description on the relationship among the distribution of drug contained in a needle, the puncture properties, the solubility, and the drug efficacy.

An object of the present invention is to provide a microneedle array which includes a sheet and needles and is capable of achieving high drug efficacy.

As the result of intensive research conducted by the present inventors in order to solve the above-described problems, it was found that the same drug efficacy as in the case of subcutaneous injection can be obtained by administering a microneedle array which includes a sheet portion and a plurality of needles present on the upper surface of the sheet portion and in which a tip portion of each needle is allowed to carry a medicament at a high concentration and the proportion of a water-soluble polymer in each needle is set such that the puncture properties to the skin become excellent, thereby completing the present invention.

In other words, according to the present invention, the following inventions are provided.

A microneedle array comprising: a sheet portion; and at least one needle present on an upper surface of the sheet portion, in which the needle contains a water-soluble polymer and a medicament, the water-soluble polymer occupies 50% by mass or greater of the total solid content of the needle, the water-soluble polymer and the medicament are present at a mass ratio of Formula 1, in a needle tip region which includes a needle tip of each needle and has a height corresponding to 20% of the total height of each needle, and the sheet portion contains a water-soluble polymer.

$$\text{Formula 1; water-soluble polymer:medicament} = 0.05 \text{ to } 0.9:1$$

A method of producing the microneedle array as defined above, comprising: a step of forming some needles by drying a mold for forming needles, filled with a first water-soluble polymer-dissolved solution containing a medicament; and a step of filling the upper surface of some needles formed as described above with a second water-soluble polymer-dissolved solution and drying the surface.

According to the present invention, it is possible to provide a microneedle array which includes a sheet portion and a plurality of needles present on the sheet portion and is capable of achieving high drug efficacy.

## BRIEF DESCRIPTION OF THE DRAWINGS

Fig. 1 is a view illustrating a needle tip region which includes a needle tip of each needle and has a height corresponding to 20% of the total height of each needle.
Fig. 2A is a perspective view illustrating a conical microneedle, Fig. 2B is a perspective view illustrating a pyramidal microneedle, and Fig. 2C is a cross-sectional view illustrating a conical and pyramidal microneedle.
Fig. 3 is a perspective view illustrating a microneedle in another shape.
Fig. 4 is a perspective view illustrating a microneedle in another shape.
Fig. 5 is a cross-sectional view of the microneedles illustrated in Figs. 3 and 4.
Fig. 6 is a perspective view illustrating a microneedle in another shape.
Fig. 7 is a perspective view illustrating a microneedle in another shape.
Fig. 8 is a cross-sectional view of the microneedles illustrated in Figs. 6 and 7.
Fig. 9 is a cross-sectional view of a microneedle in another shape in which the inclination (angle) of the side surface of the needle is continuously changed.
Figs. 10A to 10C are process views illustrating a method of producing a mold.
Fig. 11 is an enlarged view of a mold.
Fig. 12 is a cross-sectional view illustrating a mold in another shape.
Figs. 13A to 13C are views schematically illustrating a process of filling the mold with a polymer-dissolved solution containing a medicament.
Fig. 14 is a perspective view illustrating the tip of a nozzle.
Fig. 15 is a partially enlarged view of the tip of the nozzle and the mold during filling.
Fig. 16 is a partially enlarged view of the tip of the nozzle and the mold during transfer.
Figs. 17A to 17D are views describing a step of forming another microneedle array.
Figs. 18A to 18C are views describing a step of forming another microneedle array.

Fig. 19 is a view describing a peeling step.

Fig. 20 is a view describing another peeling step.

Fig. 21 is a view describing a microneedle array.

Figs. 22A and 22B are respectively a plan view and a side view of an original plate.

Fig. 23 is a view schematically illustrating a filling device used in examples.

## DESCRIPTION OF THE PREFERRED EMBODIMENTS

Hereinafter, embodiments of the present invention will be described in detail.

In the present specification, the expression "containing a predetermined amount of medicament" means that a medicament having an amount enough to exhibit drug efficacy is contained in a case where the body surface is punctured. The expression "not containing a predetermined amount of medicament" means that a medicament having an amount enough to exhibit drug efficacy is not contained, and the range of the amount of the medicament covers from a case where the medicament is not contained at all to a case where the amount thereof is not enough to exhibit the drug efficacy.

Hereinafter, "containing a predetermined amount of medicament" is expressed as "containing a medicament" and "not containing a predetermined amount of medicament" is expressed as "not containing a medicament".

In a microneedle array of the present invention, the puncture properties are improved by increasing the proportion of a water-soluble polymer in a needle and the drug efficacy is improved by increasing the proportion of a medicament in a needle tip region including a needle tip. According to the microneedle array of the present invention which has the above-described configuration, it is possible to achieve the same drug efficacy as in a case of the subcutaneous injection. Typically, the proportion of a water-soluble polymer in a needle is increased for the purpose of improving the puncture properties. However, in a case where the proportion of the water-soluble polymer in a needle is increased, the proportion of the medicament is decreased so that the drug efficacy cannot be sufficiently achieved. In the present invention, distribution of the medicament is examined by dividing a needle into a needle tip region which includes a needle tip and has a height corresponding to 20% of the total height of the needle and a region other than the region described above. Further, in the present invention, both of excellent puncture properties and high drug efficacy are achieved by increasing the proportion of the water-soluble polymer in the entire needle while increasing the proportion of the medicament in the needle tip region described above. As shown in the comparative examples in the present specification, there is a case where excellent drug efficacy cannot be achieved even through the puncture properties and the solubility are excellent. That is, a factor other than the puncture properties and the solubility is suggested for exhibition of excellent drug efficacy. For example, it is expected that high drug efficacy is not exhibited in a case where a sufficient amount of medicament is not transferred into the blood.

The achievement of high drug efficacy together with excellent puncture properties and excellent solubility by employing the above-described configuration of the present invention is an effect that cannot be expected at all from the past.

[Configuration of microneedle array]

The microneedle array of the present invention is a microneedle array including: a sheet portion; and a plurality of needles present on an upper surface of the sheet portion, in which the needle contains a water-soluble polymer and a medicament, the water-soluble polymer occupies 50% by mass or greater of the total solid content of the needle, the water-soluble polymer and the medicament are present at a mass ratio of Formula 1, in a needle tip region which includes a needle tip of each needle and has a height corresponding to 20% of the total height of each needle, and the sheet portion contains a water-soluble polymer.

$$\text{Formula 1; water-soluble polymer:medicament} = 0.05 \text{ to } 0.9{:}1$$

In the present invention, plural means one or more.

The microneedle array of the present invention includes at least a sheet portion and needles and the medicament is carried by the needles in order to efficiently administer the medicament into the skin.

The microneedle array of the present invention is a device in which a plurality of needles are arranged in an array on the upper surface side of the sheet portion. It is preferable that the needles are arranged on the upper surface side of the sheet portion. The needles may be directly arranged on the upper surface of the sheet portion or may be arranged on the upper surfaces of frustums disposed on the upper surface of the sheet portion.

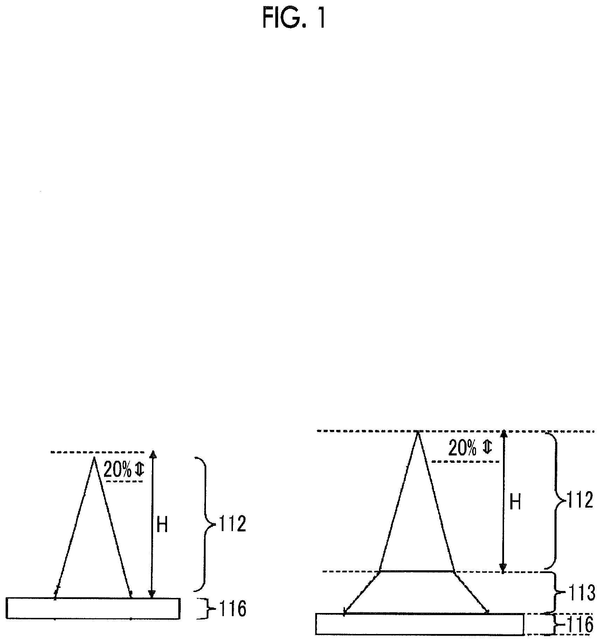

The sheet portion is a foundation for supporting needles and has a planar shape similar to the shape of a sheet portion 116 illustrated in Figs. 1 to 9. At this time, the upper surface of the sheet portion indicates the surface on which a plurality of needles are arranged in an array.

The area of the sheet portion is not particularly limited, but is preferably in a range of 0.005 to 1000 mm$^2$, more preferably in a range of 0.05 to 500 mm$^2$, and still more preferably in a range of 0.1 to 400 mm$^2$.

The thickness of the sheet portion is a distance between the surface in contact with frustums or needles and the surface on the opposite side. The thickness of the sheet portion is preferably in a range of 1 μm to 2000 μm, more preferably in a range of 3 μm to 1500 μm, and still more preferably in a range of 5 μm to 1000 μm.

The sheet portion contains a water-soluble polymer. The sheet portion may be formed of a water-soluble polymer or may contain other additives (for example, disaccharides). Further, it is preferable that the sheet portion does not contain a medicament.

The water-soluble polymer contained in the sheet portion is not particularly limited, and examples thereof include polysaccharides (such as hyaluronic acid, sodium hyaluronate, pullulan, dextran, dextrin, sodium chondroitin sulfate, carboxymethyl cellulose, hydroxypropyl cellulose, hydroxyethyl starch, hydroxypropyl methyl cellulose, polyvinylpyrrolidone, polyoxyethylene polyoxypropylene glycol, polyethylene glycol, and gum Arabic); and proteins (such as gelatin). The above-described components may be used alone or in combination of two or more kinds thereof. Among these, polysaccharides are preferable; hydroxyethyl starch, hydroxypropyl cellulose, hydroxypropyl methyl cellulose, pullulan, dextran, sodium chondroitin sulfate, sodium hyaluronate, carboxymethyl cellulose, polyvinylpyrrolidone, polyoxyethylene polyoxypropylene glycol, polyethylene glycol, and polyvinyl alcohol are more preferable; and chondroitin sulfate is particularly preferable.

Disaccharides may be added to the sheet portion and examples of the disaccharides include sucrose, lactulose, lactose, maltose, trehalose, and cellobiose. Among these, sucrose, maltose, and trehalose are particularly preferable.

The microneedle array is configured of a plurality of needles arranged in an array on the upper surface of the sheet portion. The needles have a projected structure with a tip, and the shape thereof is not limited to a needle shape having a sharp tip and may be a shape with a blunt tip.

Examples of the shape of a needle include a conical shape, a polygonal pyramid shape (square pyramid shape or the like), and a spindle shape. For example, a needle may have a shape of a needle 112 illustrated in Figs. 1 to 9, in which the entire shape of the needle may be a conical shape, a polygonal pyramid shape (square pyramid shape or the like), or a shape of a structure in which the inclination (angle) of the side surface of the needle is continuously changed. Further, a needle may have a multilayer structure with two or more layers, in which the inclination (angle) of the side surface of the needle is discontinuously changed.

In a case where the microneedle array of the present invention is applied to the skin, it is preferable that the needles are inserted into the skin and the upper surface or a part of the sheet portion is brought into contact with the skin.

The height (length) of a needle indicates the length of a perpendicular line drawn from the tip of the needle to a frustum or the sheet portion (in a case where a frustum is not present). The height (length) of a needle is not particularly limited, but is preferably in a range of 50 μm to 3000 μm, more preferably in a range of 100 μm to 1500 μm, and still more preferably in a range of 100 μm to 1000 μm. It is preferable that the length of a needle is 50 μm or greater because a medicament can be percutaneously administered. Further, it is preferable that the length of a needle is 3000 μm or less because occurrence of pain resulting from the contact of needles with the nerve is prevented and bleeding can be avoided.

The interface between a frustum (or a needle in a case where a frustum is not present) and the sheet portion is referred to as a base. The distance between a base of one needle and a point farthest from the base is preferably in a range of 50 μm to 2000 μm, more preferably in a range of 100 μm to 1500 μm, and still more preferably in a range of 200 μm to 1000 μm.

The number of needles to be arranged in one microneedle array is preferably in a range of 1 to 2000, more preferably in a range of 3 to 1000, and still more preferably in a range of 5 to 500. In a case where one microneedle array includes two needles, the interval between needles indicates the distance between feet of each perpendicular line drawn from the tip of a needle to a frustum or the sheet portion (in the case where a frustum is not present). In a case where one microneedle array includes three or more needles, the interval between needles to be arranged indicates an average value obtained by acquiring the distance between a foot of a perpendicular line drawn from the tip of a needle to a frustum or the sheet portion (in the case where frustums are not present) and a foot of a perpendicular line drawn from the tip of a needle closest to the needle to a frustum or the sheet portion and averaging the values obtained from all needles. The interval between needles is preferably in a range of 0.1 mm to 10 mm, more preferably in a range of 0.2 mm to 5 mm, and still more preferably in a range of 0.3 mm to 3 mm.

The needles contain a water-soluble polymer and a medicament.

It is preferable that the water-soluble polymer is a biosoluble substance such that a human body is not damaged even in a case where needles remain in the skin.

The water-soluble polymer contained in the needles is not particularly limited, and examples thereof include polysaccharides (such as hyaluronic acid, sodium hyaluronate, pullulan, dextran, dextrin, sodium chondroitin sulfate, carboxymethyl cellulose, hydroxypropyl cellulose, hydroxyethyl starch, hydroxypropyl methyl cellulose, polyvinylpyrrolidone, polyoxyethylene polyoxypropylene glycol, polyethylene glycol, and gum Arabic); and proteins (such as gelatin). The above-described components may be used alone or in combination of two or more kinds thereof. Among these, polysaccharides are preferable; hydroxyethyl starch, hydroxypropyl cellulose, hydroxypropyl methyl cellulose, pullulan, dextran, sodium chondroitin sulfate, sodium hyaluronate, carboxymethyl cellulose, polyvinylpyrrolidone, polyoxyethylene polyoxypropylene glycol, polyethylene glycol, and polyvinyl alcohol are more preferable; and hydroxyethyl starch is particularly preferable. Further, polysaccharides typically with no charge are more preferable because polysaccharides are unlikely to be aggregated at the time of being mixed with a medicament. The water-soluble polymer contained in the needles may be the same as or different from the water-soluble polymer contained in the sheet portion.

Disaccharides may be added to the needles (particularly, the needle tip region) and examples of the disaccharides include sucrose, lactulose, lactose, maltose, trehalose, and cellobiose. Among these, sucrose, maltose, and trehalose are preferable.

It is preferable that the needles contain two or more water-soluble polymers, disaccharides, and a medicament. Two or more water-soluble polymers are not particularly limited, but a combination of hydroxyethyl starch and other water-soluble polymers is preferable.

In the present invention, the content of the water-soluble polymer is 50% by mass or greater with respect to the total solid content of the needles. The content thereof is preferably 55% by mass or greater, more preferably 60% by mass or greater, and still more preferably 65% by mass or greater with respect to the total solid content of the needles.

The upper limit thereof is not particularly limited, but the content of the water-soluble polymer is preferably 99% by mass or less, more preferably 95% by mass or less, and still more preferably 90% by mass or less with respect to the total solid content of the needles.

In a case where the content of the water-soluble polymer is set to be 50% by mass or greater with respect to the total solid content of the needles, excellent puncture properties and excellent drug efficacy can be obtained.

The proportion of the water-soluble polymer in the total solid content of the needles can be measured using the following method, but the method of measuring the proportion thereof is not particularly limited. As an example of the measurement method, needles of a prepared microneedle array are cut, the needles are dissolved in a buffer solution (a buffer solution suitable for dissolving the water-soluble polymer constituting the needles, such as phosphate buffered saline (PBS)), and then the amount of the water-soluble polymer in the solution can be measured according to a high performance liquid chromatography method.

In the present invention, the water-soluble polymer and the medicament are present at a mass ratio of Formula 1, in a needle tip region which includes a needle tip of each needle and has a height corresponding to 20% of the total height of each needle.

Formula 1; water-soluble polymer:medicament = 0.05 to 0.9:1

The needle tip region is a partial region of a needle that includes a needle tip. As illustrated in Fig. 1, the needle tip region is a region having a height corresponding to 20% of the total height of a needle.

The left figure in Fig. 1 illustrates a case where the needle 112 is formed on the surface of the sheet portion 116. The right figure in Fig. 1 illustrates a case where a frustum 113 is formed on the surface of the sheet portion 116 and the needle 112 is formed on the upper surface of the frustum 113. In the figures, H represents the height of the needle 112 (height of the entire needle). In Fig. 1, the region which includes the needle tip and has a height corresponding to 20% of the total height of the needle is shown as "20%".

In a case where the water-soluble polymer and the medicament are present at a mass ratio of Formula 1 in the needle tip region, excellent drug efficacy can be exhibited.

In the needle tip region, the water-soluble polymer and the medicament are present preferably at a mass ratio of Formula 1A, more preferably at a mass ratio of Formula 1B, still more preferably at a mass ratio of Formula 1C, and even still more preferably at a mass ratio of Formula 1D.

Formula 1A; water-soluble polymer:medicament = 0.1 to 0.8:1

Formula 1B; water-soluble polymer:medicament = 0.1 to 0.7:1

Formula 1C; water-soluble polymer:medicament = 0.2 to 0.7:1

## Formula 1D; water-soluble polymer:medicament = 0.3 to 0.6:1

Further, in a case where the needles contain a water-soluble polymer, disaccharides, and a medicament, it is preferable that the water-soluble polymer, the disaccharides, and the medicament are present at a mass ratio of Formula 2 in the needle tip region which includes a needle tip and has a height corresponding to 20% of the total height of the needle.

## Formula 2; water-soluble polymer + disaccharides:medicament = 0.05 to 5:1

In a case where the water-soluble polymer, the disaccharides, and the medicament are present at a mass ratio of Formula 2 in the needle tip region, excellent drug efficacy can be achieved through excellent puncture properties and excellent solubility.

In the needle tip region, the water-soluble polymer, the disaccharides, and the medicament are present more preferably at a mass ratio of Formula 2A, more preferably at a mass ratio of Formula 2B, still more preferably at a mass ratio of Formula 2C, and particularly preferably at a mass ratio of Formula 2D.

## Formula 2A; water-soluble polymer + disaccharides:medicament = 0.1 to 5:1

## Formula 2B; water-soluble polymer + disaccharides:medicament = 0.1 to 4:1

## Formula 2C; water-soluble polymer + disaccharides:medicament = 0.2 to 4:1

## Formula 2D; water-soluble polymer + disaccharides:medicament = 0.3 to 3:1

The mass ratio between the water-soluble polymer and the medicament in the needle tip region and the mass ratio among the water-soluble polymer, the disaccharides, and the medicament in the needle tip region can be measured using the following method, but the method thereof is not particularly limited.

As an example of the method of measuring the mass ratio, the needle tip region which includes the needle tip and has a height corresponding to 20% of the total height of the needle is cut in parallel with the sheet portion, and the cut needle tip region is dissolved in a buffer solution (a buffer solution suitable for dissolving the water-soluble polymer and medicament constituting the needles, such as phosphate buffered saline (PBS)). The amounts of the water-soluble polymer and the disaccharides can be respectively measured according to a high performance liquid chromatography method by measuring the amount of medicament in the solution using an enzyme-linked immunosorbent assay (ELISA) method or the like.

The medicament indicates a substance that affects a human body. It is preferable that the medicament is selected from peptides (including peptide hormones or the like) or derivatives thereof, proteins, a nucleic acid, polysaccharides, vaccines, adjuvants, a pharmaceutical compound belonging to a water-soluble low molecular compound, or cosmetic ingredients. The molecular weight of the medicament is not particularly limited, but a medicament having a molecular weight of 500 or greater is preferable in a case of proteins.

Examples of peptides or derivatives thereof and proteins include calcitonin, adrenocorticotropic hormone, parathyroid hormone (PTH), human PTH (1 → 34), insulin, exendin, secretin, oxytocin, angiotensin, β-endorphin, glucagon, vasopressin, somatostatin, gastrin, luteinizing hormone releasing hormone, enkephalin, neurotensin, atrial natriuretic peptide, growth hormone, growth hormone releasing hormone, bradykinin, substance P, dynorphin, thyroid stimulating hormone, prolactin, interferon, interleukin, granulocyte colony stimulating factor (G-CSF), glutathione peroxidase, superoxide dismutase, desmopressin, somatomedin, endothelin, and salts of these.

Examples of the vaccines include influenza antigen (influenza vaccine), hepatitis B virus surface antigen (HBs) antigen, hepatitis Be antigen (HBe antigen), Bacille de calmette et Gaerin (BCG) antigen, measles antigen, rubella antigen, varicella antigen, yellow fever antigen, shingles antigen, rotavirus antigen, influenza bacilli b type (Hib) antigen, rabies antigen, cholera antigen, diphtheria antigen, pertussis antigen, tetanus antigen, inactivated polio antigen, Japanese encephalitis antigen, human papilloma antigen, and antigens obtained by mixing two to four types of these.

Examples of the adjuvants include aluminum salts such as aluminum phosphate, aluminum chloride, and aluminum hydroxide, emulsions such as MF59 and AS03, liposomes, plant-derived components, a nucleic acid, biopolymers, cytokine, peptides, proteins, and sugar chains.

Among these, as the medicament, at least one selected from the group consisting of peptide hormones, vaccines, and adjuvants is preferable. Growth hormone is particularly preferable as peptide hormones and influenza vaccine is particularly preferable as vaccines.

The content of the medicament in all needles is not particularly limited, but is preferably in a range of 1 to 60% by mass, more preferably in a range of 1 to 50% by mass, and particularly preferably in a range of 1 to 45% by mass with respect to the mass of the solid content of needles.

Hereinafter, preferred embodiments of the present invention will be described with reference to the accompanying drawings, but the present invention is not limited thereto.

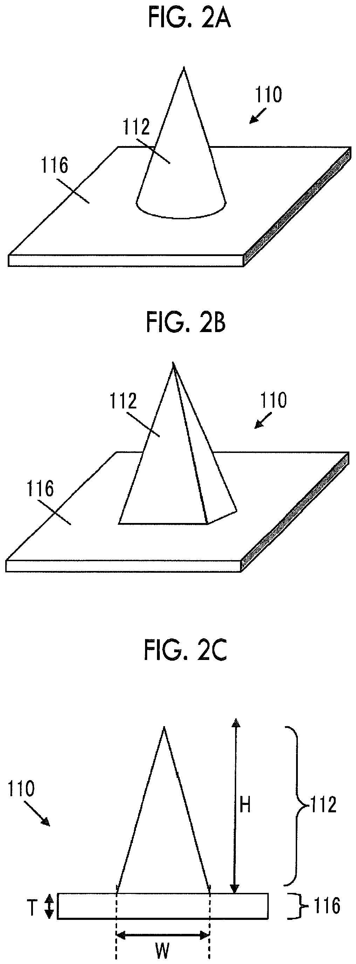

Figs. 2 to 9 are partially enlarged views illustrating a microneedle 110 in the microneedle array. The microneedle array of the present invention is configured by the plurality of needles 112 being formed on the surface of the sheet portion 116 (in the figured, one needle 112 is shown on the sheet portion 116 or one frustum 113 and one needle 112 are shown on the sheet portion 116 and this is referred to as the microneedle 110).

The needle 112 has a conical shape in Fig. 2A and the needle 112 has a square pyramid shape in Fig. 2B. In Fig. 2C, H represents the height of the needle 112, W represents the diameter (width) of the needle 112, and T represents the height (thickness) of the sheet portion 116.

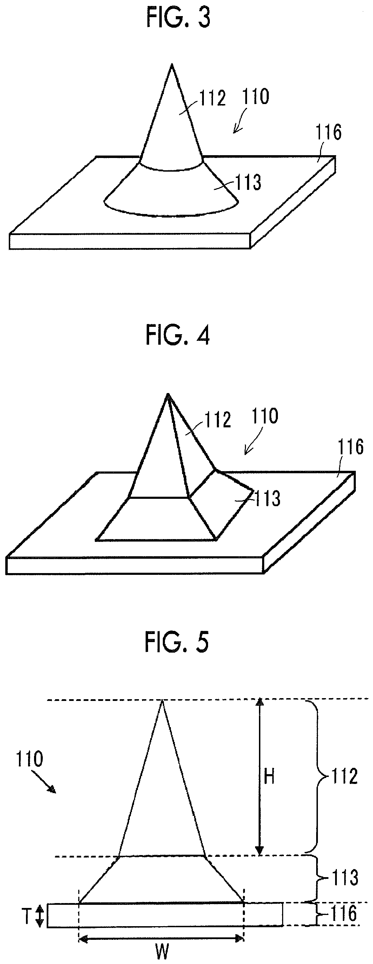

Figs. 3 and 4 illustrate microneedles 110, on which the frustum 113 and the needle 112 are formed and which have different shapes, formed on the surface of the sheet portion 116. In Fig. 3, the frustum 113 has a truncated conical shape and the needle 112 has a conical shape. In Fig. 4, the frustum 113 has a truncated square pyramid shape and the needle 112 has a square pyramid shape. However, the shape of the needle is not particularly limited.

Fig. 5 is a cross-sectional view illustrating the microneedles 110 illustrated in Figs. 3 and 4. In Fig. 5, H represents the height of the needle 112, W represents the diameter (width) of the base, and T represents the height (thickness) of the sheet portion 116.

It is preferable that the microneedle array of the present invention has a shape of the microneedle 110 of Fig. 5 other than the shape of the microneedle 110 in Fig. 2C. With such a configuration, the volume of all needles becomes larger so that a greater amount of medicament can be concentrated on the tip of a needle when the microneedle array is produced.

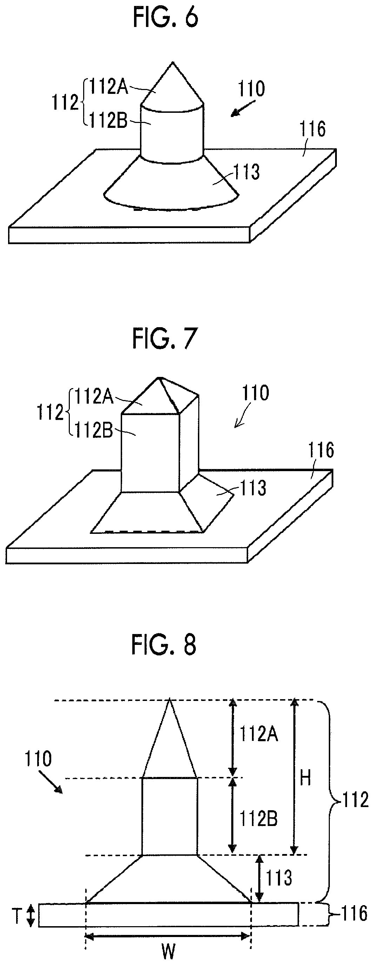

Figs. 6 and 7 illustrate microneedles 110 in different shapes.

A first layer 112A of the needle illustrated in Fig. 6 has a conical shape and a second layer 112B of the needle in Fig. 6 has a columnar shape. The first layer 112A of the needle illustrated in Fig. 7 has a square pyramid shape and the second layer 112B of the needle in Fig. 7 has a square columnar shape. However, the shape of a needle is not limited to these shapes.

Fig. 8 is a cross-sectional view illustrating the microneedles 110 illustrated in Figs. 6 and 7. In Fig. 8, H represents the height of the needle 112, W represents the diameter (width) of the base, and T represents the height (thickness) of the sheet portion 116.

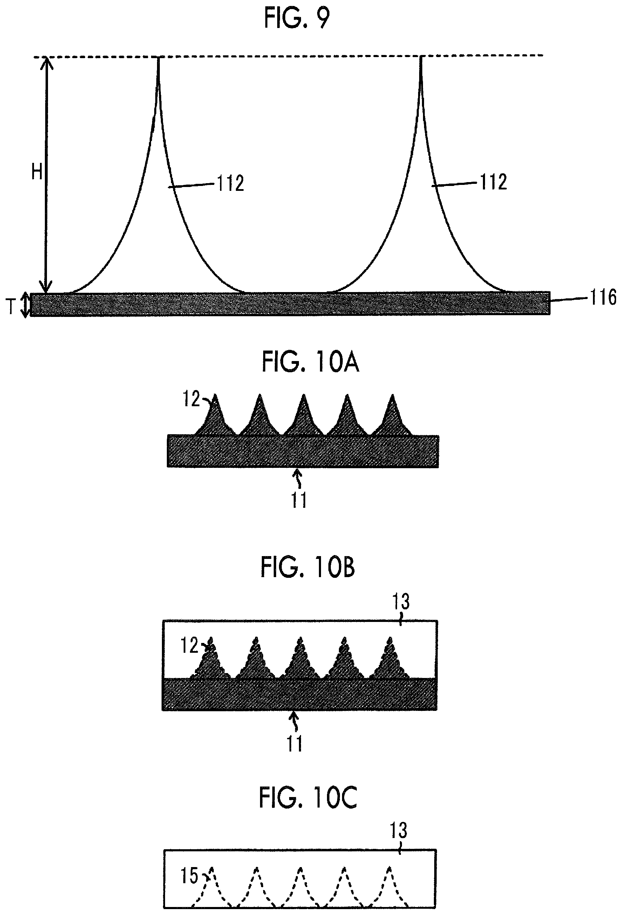

Fig. 9 is a cross-sectional view of a microneedle in another shape in which the inclination (angle) of the side surface of the needle 112 is continuously changed. In Fig. 9, H represents the height of the needle 112 and T represents the height (thickness) of the sheet portion 116.

In the microneedle array of the present invention, it is preferable that needles are arranged at intervals of approximately 0.1 to 10 needles per 1 mm in a row. It is more preferable that the microneedle array has 1 to 10000 microneedles per 1 $cm^2$. When the density of microneedles is set to 1 needle/$cm^2$ or greater, the microneedles can efficiently puncture the skin. When the density of the microneedles is set to 10000 needles/$cm^2$ or less, the microneedle array can sufficiently puncture the skin. The density of needles is preferably in a range of 10 to 5000 needles/$cm^2$, more preferably in a range of 25 to 1000 needles/$cm^2$, and particularly preferably in a range of 25 to 400 needles/$cm^2$.

The microneedle array of the present invention can be supplied in a sealed storage form together with a drying agent. As the drying agent, known drying agents (such as silica gel, calcined lime, calcium chloride, silica alumina, and a sheet-like drying agent) can be used.

[Method of producing microneedle array]

The microneedle array of the present invention can be produced by the following method in conformity with the method described in, for example, JP2013-153866A or WO2014/077242A.

(Preparation of mold)

Figs. 10A to 10C are process views illustrating a method of preparing a mold (die). As illustrated in Fig. 10A, first, an original plate is prepared used to prepare the mold. There are two methods for preparing an original plate 11.

According to the first method, a Si substrate is coated with a photoresist, exposed, and then developed. Further, an array of shaped portions 12 having a conical shape (projection) is prepared on the surface of the original plate 11 by performing etching using reactive ion etching (RIE) or the like. In addition, when the etching such as RIE or the like is performed so as to form shaped portions having a conical shape on the surface of the original plate 11, the portions having a conical shape can be formed by performing etching in an oblique direction while the Si substrate rotate. According to the second method, an array of the shaped portions 12 having a square pyramid shape or the like is formed on the surface of the original plate 11 by performing processing on a metal substrate such as Ni using a cutting tool such as a diamond bit.

Next, a mold is prepared. Specifically, a mold 13 is prepared using the original plate 11 as illustrated in Fig. 10B. As the method of preparing the mold, four methods are considered.

According to the first method, a silicone resin obtained by adding a curing agent to polydimethylsiloxane (PDMS, for example, SYLGARD 184 (registered trade mark, manufactured by Dow Corning Toray Co., Ltd.)) is poured into the original plate 11, subjected to a heat treatment at 100°C, cured, and peeled from the original plate 11. According to the second method, an ultraviolet (UV) cured resin which is cured by being irradiated with ultraviolet rays is poured into the original plate 11, irradiated with ultraviolet rays in a nitrogen atmosphere, and peeled off from the original plate 11. According to the third method, a solution obtained by dissolving a plastic resin such as polystyrene or polymethyl methacrylate (PMMA) in an organic solvent is poured into the original plate 11 coated with a peeling agent, dried so that the organic solvent is volatilized, and cured, and then peeled off from the original plate 11. According to the fourth method, an inverted product is produced using Ni electroforming.

In this manner, the mold 13 formed by needle-like recesses 15, which have an inverted shape of the conical shape or the pyramid shape of the original plate 11, being two-dimensionally arranged is prepared. The mold 13 prepared in the above-described manner is illustrated in Fig. 10C.

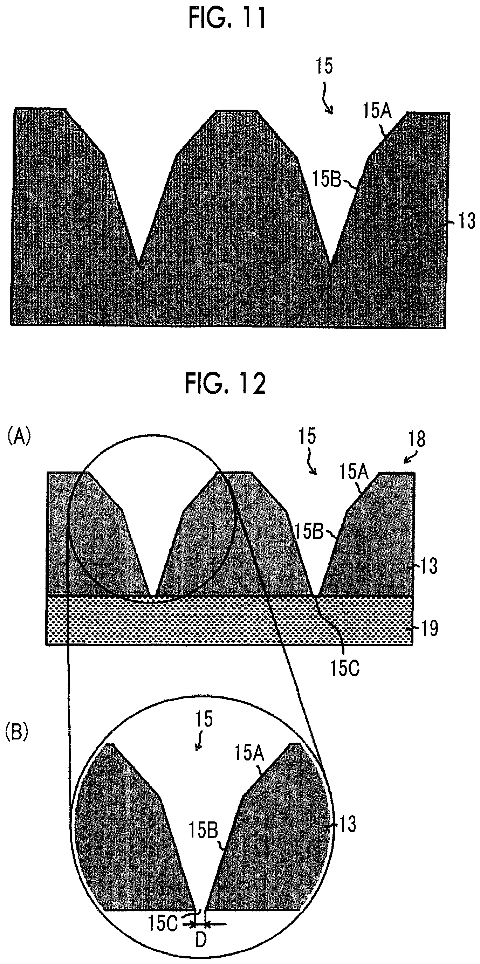

Fig. 11 illustrates another preferred embodiment of the mold 13. The needle-like recess 15 includes a tapered inlet portion 15A which becomes narrower in a depth direction from the surface of the mold 13 and a tip recess 15B which becomes tapered in the depth direction. When the inlet portion 15A has a tapered shape, the needle-like recess 15 is easily filled with the water-soluble polymer-dissolved solution.

Fig. 12 illustrates a more preferred embodiment of a mold complex 18 at the time of producing the microneedle array. The (A) portion of Fig. 12 illustrates the mold complex 18. The (B) portion of Fig. 12 is a partially enlarged view of a portion enclosed by a circle in the (A) portion.

As illustrated in the (A) portion of Fig. 12, the mold complex 18 includes the mold 13 having an air vent hole 15C formed on the tip (bottom) of the needle-like recess 15; and an air permeating sheet 19 which is bonded to the rear surface of the mold 13 and is formed of a material that permeates a gas and does not permeate a liquid. The air vent hole 15C is formed as a through-hole penetrating the rear surface of the mold 13. Here, the rear surface of the mold 13 indicates the surface on a side on which the air vent hole 15C is formed. With this configuration, the tip of the needle-like recess 15 communicates with the air through the air vent hole 15C and the air permeating sheet 19.

When such a mold complex 18 is used, only the air present in the needle-like recess 15 can be released from the needle-like recess 15 without permeation of the polymer-dissolved solution filling the needle-like recess 15. In this manner, the property of transferring the shape of the needle-like recess 15 to a polymer becomes excellent in the above-described manner and a sharper needle can be formed.

A diameter D (diameter) of the air vent hole 15C is preferably in a range of 1 to 50 $\mu$m. In a case where the diameter D of the air vent hole 15C is less than 1 $\mu$m, the air vent hole 15C cannot be sufficiently used as an air bend hole. Further, in a case where the diameter D of the air vent hole 15C is greater than 50 $\mu$m, the sharpness of the tip of a formed microneedle is damaged.

As the air permeating sheet 19 formed of a material that permeates a gas and does not permeate a liquid, for example, an air permeating film (Poreflon (registered trade mart), FP-010, manufactured by Sumitomo Electric Industries, Ltd.) can be suitably used.

As the material used for the mold 13, an elastic material or a metal material can be used. Among these, an elastic material is preferable and a material having a high gas permeability is more preferable. The oxygen permeability, which is a representative example of the gas permeability, is preferably $1 \times 10^{-12}$ (mL/s·m²·Pa) or greater and more preferably $1 \times 10^{-10}$ (mL/s·m²·Pa) or greater. Further, 1 mL is $10^{-6}$ m³. When the gas permeability is in the above-described range, the air present in a recess of the mold 13 can be released from the die and a microneedle array with less defects can be produced. Specific examples of such materials include materials obtained by melting or dissolving, in a solvent, a silicone resin (for example, SYLGARD 184 (registered trade mark, manufactured by Dow Corning Toray Co., Ltd.) or KE-1310ST (product number, manufactured by Shin-Etsu chemical Co., Ltd.)), a UV curable resin, or a plastic resin (for example, polystyrene or polymethyl methacrylate (PMMA)). Among these, a silicone rubber-based material is preferable since the material has durability to transfer resulting from repetitive pressure and has excellent peeling properties with respect to a material. Further, examples of the metal material include Ni, Cu, Cr, Mo, W, Ir, Tr, Fe, Co, MgO, Ti, Zr, Hf, V, Nb, Ta, $\alpha$-aluminum oxide, zirconium oxide, stainless (for example, STAVAX (registered trademark) of Bohler-Uddeholm KK), and alloys thereof. As the material of a frame 14, the same material as the material of the mold 13 can be used.

(Water-soluble polymer-dissolved solution)

In the present invention, it is preferable to prepare a water-soluble polymer-dissolved solution containing a medicament used to form at least a part of a needle and a water-soluble polymer-dissolved solution used to form the sheet portion.

The type of water-soluble polymer is as described in the present specification above.

Disaccharides may be mixed with both of the water-soluble polymer-dissolved solutions, and the type of disaccharides is as described in the present specification above.

The concentration of the water-soluble polymer in any of the water-soluble polymer-dissolved solutions varies depending on the type of the water-soluble polymer to be used, and is preferably in a range of 1 to 50% by mass. Further, a solvent used for dissolution may be a solvent other than hot water as long as the solvent has volatility, and methyl ethyl ketone (MEK) or alcohol can be used as the solvent.

(Formation of needle)

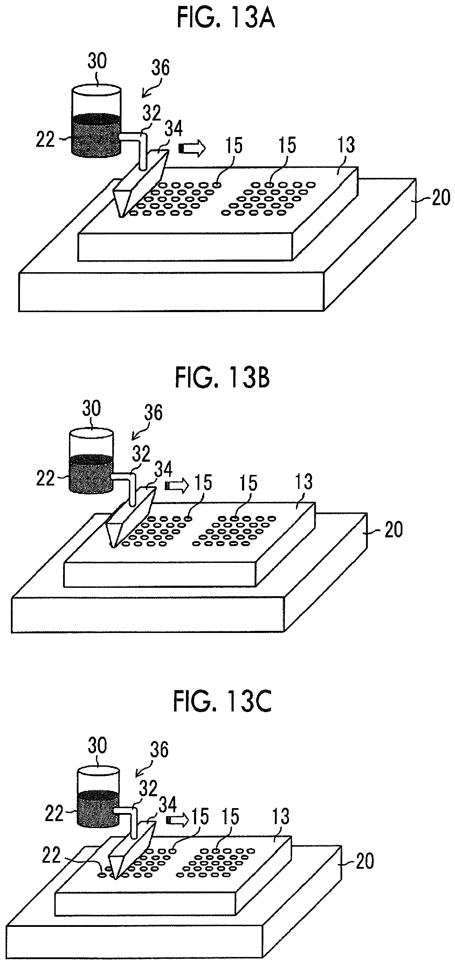

As illustrated in Fig. 13A, the mold 13 having needle-like recesses 15 which are two-dimensionally arranged is disposed on a base 20. In the mold 13, two sets of plural needle-like recesses 15 are formed such that 5 rows of needle-like recesses 15 and 5 columns of needle-like recesses 15 are two-dimensionally arranged. A liquid supply device 36 including a tank 30 which accommodates a water-soluble polymer-dissolved solution 22 containing a medicament; a pipe 32 which is connected with the tank; and a nozzle 34 which is connected with the tip of the pipe 32 is prepared. Further, in the present example, the case where 5 rows of needle-like recesses 15 and 5 columns of needle-like recesses 15 are two-dimensionally arranged is exemplified, but the number of the needle-like recesses 15 is not limited to 5 rows $\times$ 5 columns as long as the needle-like recesses are two-dimensionally arranged in a manner of M $\times$ N (M and N each independently represent an arbitrary integer of 1 or greater, preferably in a range of 2 to 30, more preferably in a range of 3 to 25, and still more preferably in a range of 3 to 20).

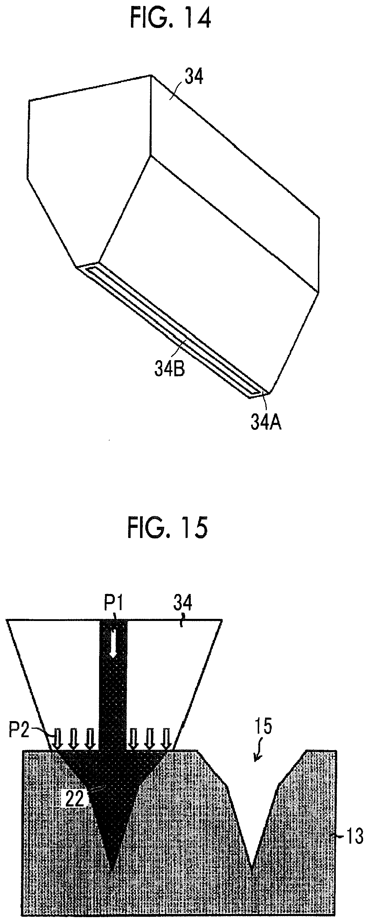

Fig. 14 is a perspective view schematically illustrating the tip portion of the nozzle. As illustrated in Fig. 14, the tip of the nozzle 34 includes a lip portion 34A which is a flat surface and an opening portion 34B having a slit shape. For example, a plurality of needle-like recesses 15 forming one row can be concurrently filled with the water-soluble polymer-dissolved solution 22 containing a medicament because of the opening portion 34B having a slit shape. The size (the length and the width) of the opening portion 34B can be suitably selected according to the number of needle-like recesses 15 to be filled with the water-soluble polymer-dissolved solution at the same time. When the length of the opening portion 34B is set to be large, a larger amount of needle-like recesses 15 can be filled with the polymer-dissolved solution 22 containing a medicament at the same time. In this manner, the productivity can be improved.

As the material used for the nozzle 34, an elastic material or a metal material can be used. Examples thereof include TEFLON (registered trademark), stainless steel (steel special use stainless (SUS)), and titanium.

As illustrated in Fig. 13B, the position of the opening portion 34B of the nozzle 34 is adjusted on the needle-like recesses 15. The lip portion 34A of the nozzle 34 is in contact with the surface of the mold 13. The water-soluble polymer-dissolved solution 22 containing a medicament is supplied to the mold 13 from a liquid supply device 36, and the water-soluble polymer-dissolved solution 22 containing a medicament fills the needle-like recesses 15 from the opening portion 34B of the nozzle 34. In the present embodiment, a plurality of needle-like recesses 15 forming one row can be concurrently filled with the water-soluble polymer-dissolved solution 22 containing a medicament. However, the present invention is not limited thereto, and the needle-like recesses 15 can be filled with the water-soluble polymer-dissolved solution one by one.

In a case where the mold 13 is formed of a material having a gas permeability, the solution 22 containing a medicament can be suctioned by suctioning the solution from the rear surface of the mold 13, and the filling of the needle-like recesses 15 with the polymer-dissolved solution 22 containing a medicament can be promoted.

Next to the filling process of Fig. 13B, as illustrated in Fig. 13C, the lip portion 34A of the nozzle 34 is brought into contact with the surface of the mold 13, the liquid supply device 36 is relatively moved in the length direction and the vertical direction of the opening portion 34B, and the nozzle 34 is moved to the needle-like recesses 15 which are not filled with the water-soluble polymer-dissolved solution 22 containing a medicament. The position of the opening portion 34B of the nozzle 34 is adjusted on the needle-like recesses 15, as illustrated in Fig. 13C. In the present embodiment, the example of moving the nozzle 34 has been described, but the mold 13 may be moved.

Since the lip portion 34A of the nozzle 34 is brought into contact with the surface of the mold 13 and then the movement is made, the water-soluble polymer-dissolved solution 22 containing a medicament, which remains on the surface other than the needle-like recesses 15 of the mold 13 can be collected by the nozzle 34. It is possible to prevent the polymer-dissolved solution 22 containing a medicament from remaining on the surface other than the needle-like recesses 15 of the mold 13.

In order to reduce the damage to the mold 13 and suppress deformation due to compression of the mold 13 as much as possible, it is preferable that the pressing pressure of the nozzle 34 against the mold 13 is set to be as small as possible during the movement. Further, in order to prevent the polymer-dissolved solution 22 containing a medicament from remaining on the surface other than the needle-like recesses 15 of the mold 13, it is preferable that at least one of the mold 13 or the nozzle 34 is formed of a flexible material which can be elastically deformed.

By repeating the filling process of Fig. 13B and the moving process of Fig. 13C, 5 rows and 5 columns of needle-like recesses 15 which are two dimensionally arranged are filled with the water-soluble polymer-dissolved solution 22 containing a medicament. When 5 rows and 5 columns of needle-like recesses 15 which are two dimensionally arranged are filled with the water-soluble polymer-dissolved solution 22 containing a medicament, the liquid supply device 36 is moved to 5 rows and 5 columns of two-dimensionally arranged needle-like recesses 15 which are adjacent to the needle-like recesses filled with the solution and then the filling process of Fig. 13B and the moving process of Fig. 13C are repeated. The 5 rows and 5 columns of two-dimensionally arranged needle-like recesses 15 which are adjacent to the needle-like recesses filled with the solution are filled with the water-soluble polymer-dissolved solution 22 containing a medicament.

For the above-described filling process and moving process, (1) embodiment in which the needle-like recesses 15 are filled with the water-soluble polymer-dissolved solution 22 containing a medicament while the nozzle 34 is moved or (2) embodiment in which the nozzle 34 is temporarily stopped on the needle-like recesses 15 during the movement of the nozzle 34, the needle-like recesses 15 are filled with the polymer-dissolved solution 22 containing a medicament, and the nozzle 34 is moved again after the filling may be adopted. The lip portion 34A of the nozzle 34 is brought into the surface of the mold 13 between the filling process and the moving process.

Fig. 15 is a partially enlarged view of the mold 13 and the tip of the nozzle 34 at the time of filling the needle-like recess 15 with the water-soluble polymer-dissolved solution 22 containing a medicament. As illustrated in Fig. 15, the filling of the needle-like recess 15 with the water-soluble polymer-dissolved solution 22 containing a medicament can be promoted by applying a pressing force P1 into the nozzle 34. Further, when the needle-like recess 15 is filled with the water-soluble polymer-dissolved solution 22 containing a medicament, it is preferable that a pressing pressure P2 for bringing the nozzle 34 into contact with the surface of the mold 13 is set to be greater than or equal to the pressing force P1 applied into the nozzle 34. When the pressing pressure P2 is set to be greater than or equal to the pressing force PI, it is possible to suppress leaking of the water-soluble polymer-dissolved solution 22 containing a medicament to the surface of the mold 13 from the needle-like recess 15.

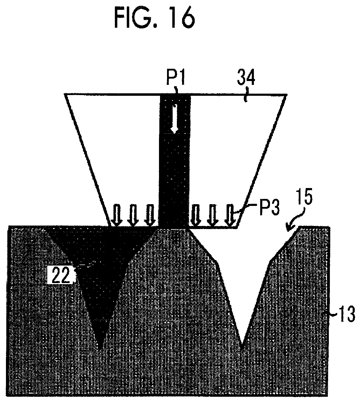

Fig. 16 is a partially enlarged view of the tip of the nozzle 34 and the mold 13 during the movement of the nozzle 34. When the nozzle 34 is relatively moved with respect to the mold 13, it is preferable that a pressing pressure P3 of bringing the nozzle 34 into contact with the surface of the mold 13 is set to be smaller than the pressing pressure P2 of bringing the nozzle 34 into contact with the surface of the mold 13 during the filling. When the pressing pressure P3 is set to be smaller than the pressing force P2, the damage to the mold 13 is reduced and the deformation of the mold 13 due to compression is suppressed.

When the filling of the plurality of needle-like recesses 15 formed of 5 rows and 5 columns of needle-like recesses is completed, the nozzle 34 is moved to the plurality of needle-like recesses 15 formed of 5 rows and 5 columns of needle-like recesses adjacent to the needle-like recesses filled with the solution. When the nozzle 34 is moved to the plurality of needle-like recesses 15 formed of 5 rows and 5 columns of needle-like recesses adjacent to the needle-like recesses filled with the solution at the time of liquid supply, it is preferable that the supply of the water-soluble polymer-dissolved solution 22 containing a medicament is stopped. There is a distance between the needle-like recesses 15 in the fifth row and the needle-like recesses 15 in the next first row. When the water-soluble polymer-dissolved solution 22 containing a medicament is continuously supplied during the movement of the nozzle 34 between the rows, the liquid pressure inside of the nozzle 34 is extremely high in some cases. As the result, the polymer-dissolved solution 22 containing a medicament supplied from the nozzle 34 occasionally flows out of the needle-like recesses 15 of the mold 13. In order to prevent the solution from flowing out, it is preferable that the liquid pressure inside the nozzle 34 is detected and the supply of the polymer-dissolved solution 22 containing a medicament is stopped when it is determined that the liquid pressure is extremely high.

In the above, the method of supplying the polymer-dissolved solution containing a medicament using a dispenser that has a nozzle has been described, but bar coating, spin coating, or spray coating can be applied in addition to the coating with the dispenser.

In the present invention, it is preferable that the drying treatment is performed after the water-soluble polymer-dissolved solution containing a medicament is supplied to the needle-like recesses.

Preferably, the microneedle array of the present invention can be produced by performing a process of forming some needles by drying a mold for forming needles, filled with the first water-soluble polymer-dissolved solution containing a medicament; and a process of filling the upper surface of some needles formed in the above-described manner with the second water-soluble polymer-dissolved solution and drying the mold.

It is preferable that the mold for forming needles, filled with the first water-soluble polymer-dissolved solution containing a medicament is dried under the condition in which the moisture content of the first water-soluble polymer-dissolved solution reaches 20% or less after 300 minutes from when 30 minutes elapse after the drying is started. Drying of the mold under the above-described conditions contributes to achieving the configuration in which "the water-soluble polymer and the medicament are present in the needle tip region at a mass ratio of Formula 1".

It is particularly preferable that the drying is controlled such that the temperature is maintained at which the medicament does not lose its effect and the moisture content of the first water-soluble polymer-dissolved solution reaches 20% or less after at least 60 minutes elapse from the start of drying.

As a method of controlling the above-described drying speed, arbitrary means capable of delaying the drying, for example, the temperature, the humidity, the drying air volume, the use of a container, or the volume and/or the shape of the container can be selected.

Preferably, the mold for forming needles, filled with the first water-soluble polymer-dissolved solution containing a medicament can be dried in a state in which the mold is covered by a container or the mold is accommodated in a container.

The temperature of drying is preferably in a range of 1°C to 45°C and more preferably in a range of 1°C to 40°C.

The relative humidity of drying is preferably in a range of 10% to 95%, more preferably in a range of 20% to 95%, and still more preferably in a range of 30% to 95%.

(Formation of sheet portion)

Several embodiments of a process of forming the sheet portion will be described.

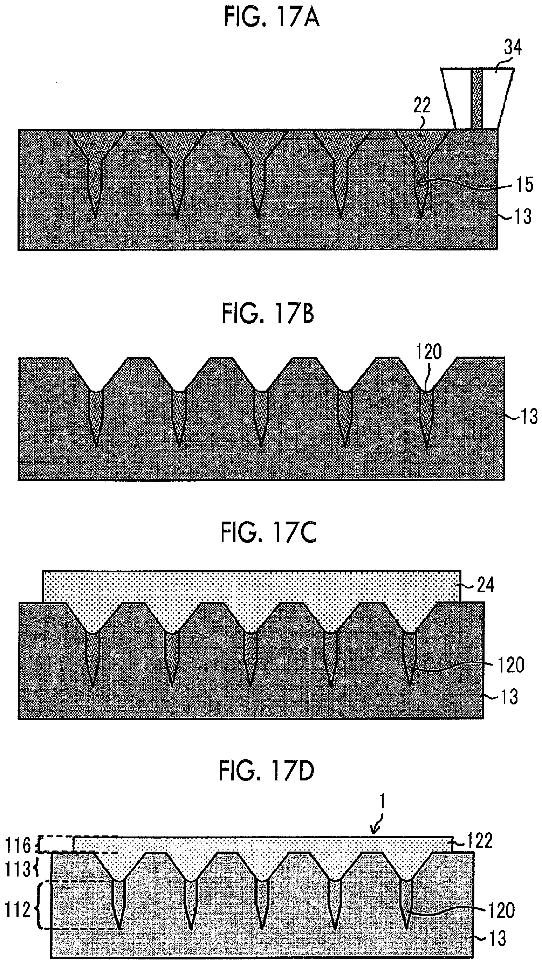

A first embodiment of a process of forming the sheet portion will be described with reference to Figs. 17A to 17D. The needle-like recesses 15 of the mold 13 are filled with the water-soluble polymer-dissolved solution 22 containing a medicament from the nozzle 34. Next, a layer 120 containing a medicament in the needle-like recesses 15 is formed by drying and solidifying the polymer-dissolved solution 22 containing a medicament as illustrated in Fig. 17B. Subsequently, the mold 13 on which the layer 120 containing a medicament is formed is coated with the water-soluble polymer-dissolved solution 24 using a dispenser as illustrated in Fig. 17C. In addition to the coating with the dispenser, bar coating, spin coating, or spray coating can be applied. Since the layer 120 containing a medicament is solidified, it is possible to prevent the medicament from being diffused in the water-soluble polymer-dissolved solution 24. Next, the microneedle array 1 including a plurality of needles 112, frustums 113, and the sheet portion 116 is formed by drying and solidifying the water-soluble polymer-dissolved solution 24 as illustrated in Fig. 17D.

In the first embodiment, in order to promote the filling of the needle-like recesses 15 with the water-soluble polymer-dissolved solution 22 and the water-soluble polymer-dissolved solution 24 containing a medicament, it is preferable to apply a pressure from the surface of the mold 13 and perform suctioning from the rear surface of the mold 13 under reduced pressure.

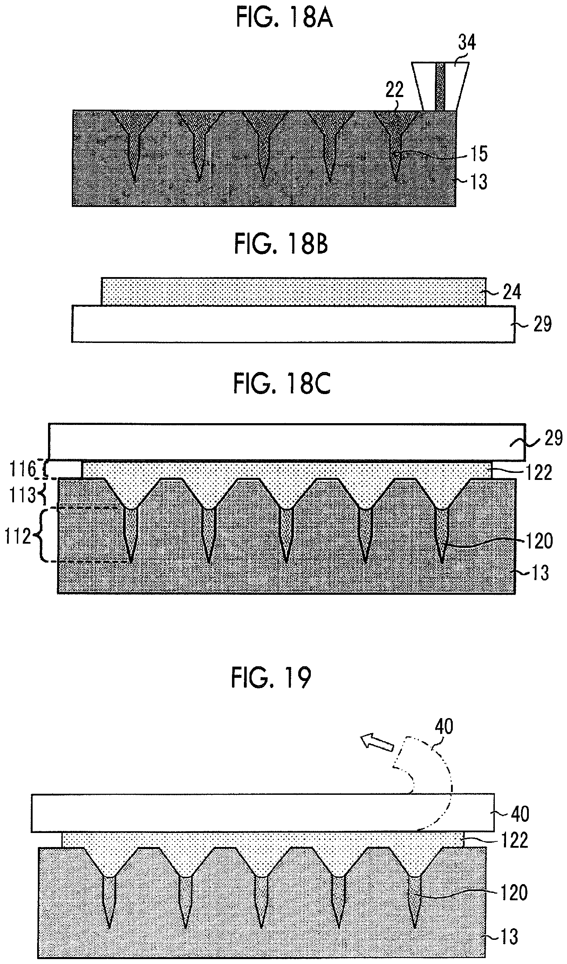

Subsequently, a second embodiment of a process will be described with reference to Figs. 18A to 18D. The needle-like recesses 15 of the mold 13 are filled with the polymer-dissolved solution 22 containing a medicament from the nozzle 34 as illustrated in Fig. 18A. Next, similar to Fig. 17B, the layer 120 containing a medicament is formed in the needle-like recesses 15 by drying and solidifying the water-soluble polymer-dissolved solution 22 containing a medicament. Next, another support 29 is coated with the water-soluble polymer-dissolved solution 24 as illustrated in Fig. 18B. The support 29 is not limited, and examples of the support include polyethylene, polyethylene terephthalate, polycarbonate, polypropylene, an acrylic resin, triacetyl cellulose, and glass. Subsequently, the water-soluble polymer-dissolved solution 24 formed on the support 29 overlaps with the mold 13 having the layer 120 containing a medicament formed on the needle-like recesses 15 as illustrated in Fig. 18C. In this manner, the needle-like recesses 15 are filled with the water-soluble polymer-dissolved solution 24. Since the layer containing a medicament is solidified, it is possible to prevent the medicament from being diffused in the water-soluble polymer-dissolved solution 24. Next, the microneedle array 1 including a plurality of needles 112, frustums 113, and the sheet portion 116 is formed by drying and solidifying the water-soluble polymer-dissolved solution 24.

In the second embodiment, in order to promote the filling of the needle-like recesses 15 with the water-soluble polymer-dissolved solution 24, it is preferable to apply a pressure from the surface of the mold 13 and perform decompressing and suctioning from the rear surface of the mold 13.

As the method of drying the water-soluble polymer-dissolved solution 24, a process of volatilizing the solvent in the polymer-dissolved solution may be exemplified. The method is not particularly limited, and a method of performing heating, blowing air, or decompression may be used. The drying treatment can be performed under the conditions of 1°C to 50°C for 1 to 72 hours. Examples of the method of blowing air include a method of blowing hot air at 0.1 to 10 m/sec. It is preferable that the drying temperature is set to a temperature at which the medicament in the polymer-dissolved solution 22 containing a medicament is not thermally deteriorated.

(Peeling)

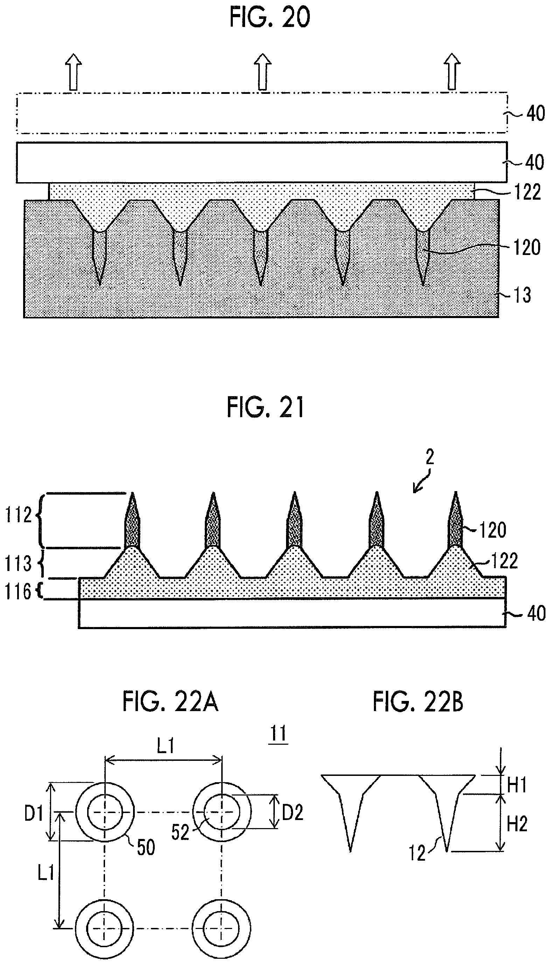

A method of peeling the microneedle array from the mold 13 is not particularly limited. It is preferable that needle projections are not bent or broken at the time of peeling. Specifically, a sheet-like base material 40 on which a pressure sensitive adhesive layer is formed is attached to the microneedle array and then the base material 40 can be peeled off from the end portion such that the base material 40 is turned over as illustrated in Fig. 19. However, the needle projections can be bent when this method is used. Therefore, as illustrated in Fig. 20, a sucking disc (not illustrated) is disposed on the base material 40 on the microneedle array so that a method of vertically pulling the base material up while suctioning the base material with air can be applied. Further, the support 29 may be used as the base material 40.

Fig. 21 illustrates the microneedle array 2 peeled from the mold 13. The microneedle array 2 includes the base material 40, the needles 112 formed on the base material 40, the frustums 113, and the sheet portion 116. At least the tip of the needle 112 has a conical shape or a polygonal pyramid shape, but the shape of the needle 112 is not limited thereto.

Hereinafter, the present invention will be described in detail with reference to examples. The materials, the amounts to be used, the ratios, the treatment contents, and the treatment procedures shown in the examples described below can be appropriately changed as long as they are within the gist of the present invention. Accordingly, the scope of the present invention should not be limitatively interpreted by the specific examples described below.

The method of producing the microneedle array of the present invention is not particularly limited, but it is preferable that the microneedle array is obtained by a production method including (1) a step of producing a mold; (2) a step of preparing a medicament and a water-soluble polymer; (3) a step of filling the mold with the liquid obtained in the step (2) and forming an upper end portion of each needle; (4) a step of filling the mold with the water-soluble polymer and forming a lower end portion of each needle and a sheet portion; and (5) a step of peeling the microneedle array from the mold.

Examples

Preparation of human growth hormone (hGH)-containing microneedle array

(Production of mold)

An original plate 11 was prepared by arranging shaped portions 12 having a needle-like structure, on which a cone 52 with a diameter D2 of 300 $\mu$m and a height H2 of 500 $\mu$m was formed, as illustrated in Fig. 22, on a truncated cone 50 having a bottom surface with a diameter D1 of 500 $\mu$m and having a height H1 of 150 $\mu$m on the surface of a smooth Ni plate in which each side had a length of 40 mm and performing grinding processing on 100 needles having a pitch L1 of 1000 $\mu$m and a square pyramid shape in a two-dimensional square array. The original plate 11 was covered by silicon rubber (SILASTIC MDX 4-4210, manufactured by Dow Corning Toray Co., Ltd.) to form a film having a thickness of 0.6 mm and the film was thermally cured in a state in which 50 $\mu$m of the conical tip of the original plate 11 protruded from the film surface and then peeled off. In this manner, an inverted product of the silicon rubber having a through-hole with a diameter of approximately 30 $\mu$m was prepared. The silicon rubber inverted product which had 10 rows and 10 columns of needle-like recesses that were two-dimensionally arranged and formed on the central portion and in which the portion other than the flat surface portion in which each side had a length of 30 mm was cut off was used as a mold. A surface on which the opening portion of a needle-like recess was wide was set to the surface of the mold and a surface having a through-hold (air vent hole) with a diameter of 30 $\mu$m was set to the rear surface of the mold.

(Preparation of water-soluble polymer-dissolved solution containing human growth hormone (hGH))

After human growth hormone (hGH) (GENOTROPIN (registered trademark), Pfizer Inc.) was concentrated by a centrifugal ultrafiltration method, an aqueous solution formed by mixing the concentrated human growth hormone with hydroxyethyl starch (HES) (Fresenius Kabi) and sucrose (Suc) (Japanese Pharmacopoeia grade, Wako Pure Chemical Industries, Ltd.) was prepared to obtain a water-soluble polymer-dissolved solution containing a medicament. Each solution is as listed in the following table.

[Table 1]

|  | hGH | HES | Suc |
|---|---|---|---|
| Comparative Example 1 | 50.00 mg/mL | 50.00 mg/mL | 0.00 mg/mL |
| Comparative Example 2 | 50.00 mg/mL | 50.00 mg/mL | 0.00 mg/mL |

(continued)

|  | hGH | HES | Suc |
|---|---|---|---|
| Comparative Example 3 | 50.00 mg/mL | 0.00 mg/mL | 0.00 mg/mL |
| Comparative Example 4 | 50.00 mg/mL | 25.00 mg/mL | 0.00 mg/mL |
| Comparative Example 5 | 50.00 mg/mL | 100.00 mg/mL | 100.00 mg/mL |
| Example 1 | 50.00 mg/mL | 45.00 mg/mL | 0.00 mg/mL |
| Example 2 | 50.00 mg/mL | 2.50 mg/mL | 0.00 mg/mL |
| Example 3 | 50.00 mg/mL | 25.00 mg/mL | 0.00 mg/mL |
| Example 4 | 50.00 mg/mL | 25.00 mg/mL | 12.50 mg/mL |
| Example 5 | 15.00 mg/mL | 7.50 mg/mL | 67.50 mg/mL |
| Example 6 | 15.00 mg/mL | 7.50 mg/mL | 75.00 mg/mL |

(Preparation of water-soluble polymer-dissolved solution forming sheet portion)

Chondroitin sulfate (manufactured by Maruha Nichiro Corporation) and disaccharides (sucrose) were dissolved in water at an arbitrary concentration ratio so as to have the composition listed in Table 2 at the time of formation of a microneedle array to prepare a water-soluble polymer-dissolved solution forming a sheet portion.

(Filling and drying of polymer-dissolved solution containing medicament)

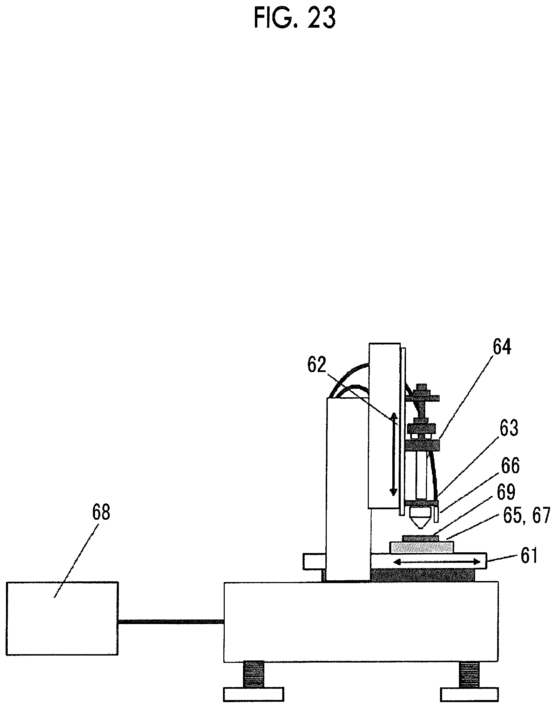

A filling device illustrated in Fig. 23 was used. The filling device includes an X-axis drive unit 61 and a Z-axis drive unit 62 which control relative position coordinates of a mold and a nozzle; a liquid supply device 64 (ultratrace determination dispenser SMP-III, manufactured by Musashi Engineering, Inc.) to which the nozzle 63 is attachable; a suction stand 65 which fixes a mold 69; a laser displacement meter 66 (HL-C201A, manufactured by Panasonic Corporation) which measures the shape of the mold surface; a load cell 67 (LCX-A-500N, manufactured by Kyowa Electronic Instruments Co., Ltd.) which measures the pressing pressure of the nozzle; and a control mechanism 68 which controls the Z-axis based on data of measured values of the surface shape and the pressing pressure.

An air permeating film (Poreflon (registered trade mart), FP-010, manufactured by Sumitomo Electric Industries, Ltd.) in which each side had a length of 15 mm was placed on a horizontal suction stand, and the mold was disposed on the surface thereof such that the surface of the mold faced up. The air permeating film and the mold were fixed to a vacuum stand by performing decompression in the rear surface direction of the mold with a suction pressure of a gauge pressure of 90 kPa.

A stainless steel (SUS) nozzle in a shape illustrated in Fig. 14 was prepared, and a slit-like opening portion having a length of 12 mm and a width of 0.2 mm was formed in the center of a lip portion having a length of 20 mm and a width of 2 mm. This nozzle was connected to a liquid supply device. The liquid supply device and the inside of the nozzle were filled with a 3 mL water-soluble polymer-dissolved solution containing a medicament. The nozzle was adjusted such that the opening portion was set to be in parallel with a plurality of needle-like recesses in the first row, formed on the surface of the mold. The nozzle was pressed to the mold at a pressure of $1.372 \times 10^4$ Pa (0.14 kgf/cm$^2$) in a position spaced by 2 mm in a direction opposite to the second row with respect to the first row. The water-soluble polymer-dissolved solution containing a medicament was allowed to be released from the opening portion at 0.15 μL/sec for 20 seconds in the liquid supply device while the nozzle was moved in the length direction and the vertical direction of the opening portion at 0.5 mm/sec in a state in which the nozzle was pressed and the Z axis was controlled such that the fluctuation in the pressing pressure was in a range of $\pm 0.490 \times 104$ Pa (0.05 kgf/cm$^2$). The movement of the nozzle was stopped in a position spaced by 2 mm after the nozzle passed through the hole pattern of the plurality of needle-like recesses two-dimensionally arranged and then the nozzle was separated from the mold.

The mold filled with the water-soluble polymer-dissolved solution containing a medicament was allowed to stand in a state of being covered by a container in an environment of a temperature of 23°C and a relative humidity of 45% and then dried. At this time, the water-soluble polymer-dissolved solution containing a medicament was gradually dried and the moisture content thereof was adjusted to 20% or less after 60 minutes elapsed.

(Forming and drying sheet portion)

As a support of forming the sheet portion, a support on which a hydrophilized plasma treatment was performed under the following conditions (used gas: $O_2$, gas pressure: 13 Pa, high frequency (RF) powder: 100 W, irradiation time: 3 minutes, $O_2$ flow rate: SV250, target vacuum degree (CCG): $2.0 \times 10^{-4}$ Pa) using a polyethylene terephthalate (PET) sheet (175 $\mu$m) and a cloud remover (Victor Jvc, Ltd.) was used. The PET subjected to the treatment was coated with the water-soluble polymer-dissolved solution such that the front and rear surfaces had a film thickness of 75 $\mu$m. Further, the mold filled with the polymer-dissolved solution containing a medicament was suctioned and fixed to the suction stand. The surface of the PET coated with the water-soluble polymer-dissolved solution was disposed to face the surface of the mold and the interval between the PET and the mold and the interval between the PET and the space on a side opposite to the mold were decompressed for 2 minutes. After the decompression, the PET coated with the water-soluble polymer-dissolved solution and the mold were bonded to each other by releasing the atmospheric pressure only in the interval between the PET and the space on a side opposite to the mold. The resultant formed by the PET and the mold being bonded to each other and being integrated with each other was dried after the state in which the PET was in contact with the mold was maintained for 10 minutes.

(Peeling)

The dried and solidified microneedle array was carefully peeled off from the mold to form a microneedle array containing human growth hormone. The microneedle array includes frustums and needles. The length of a needle is approximately 460 $\mu$m and the width of a base portion of a needle is approximately 270 $\mu$m. The frustum has a truncated cone structure, the height of the frustum is approximately 130 $\mu$m, the diameter of the upper bottom surface of the frustum is approximately 270 $\mu$m, and the diameter of the lower bottom surface of the frustum is approximately 460 $\mu$m. The thickness of the sheet portion is approximately 205 $\mu$m (the thickness of polyethylene terephthalate is approximately 175 $\mu$m). The number of needles is 100, the interval between needles is approximately 1 mm, and the needles are arranged in the square form.

Distribution of needles of human growth hormone (hGH)-containing microneedle array

The interface between needles and frustums of the prepared microneedle array was cut in parallel with the sheet portion, and the needles were immersed in a buffer solution so as to be dissolved therein. The amount of hGH in the solution was measured using an enzyme-linked immunosorbent assay (ELISA) method, and the amount of HES, the amount of Suc, and the amount of chondroitin sulfate (CS) were respectively measured according to a high performance liquid chromatography method.

Next, the needle tip region including a needle tip of a needle and having a height corresponding to 20% of the total height of the needle was cut in parallel with the sheet portion, and the cut needle tip region was dissolved in a buffer solution. The amount of hGH in the solution was measured using an enzyme-linked immunosorbent assay (ELISA) method, and the amount of HES, the amount of Suc, and the amount of CS were respectively measured according to a high performance liquid chromatography method. In this manner, it was confirmed that the microneedle array listed in Table 2 was obtained.

Evaluation of puncture properties of human growth hormone (hGH)-containing microneedle array

After extracted pig skin (Yucatan Micropig Skin Set, Sinclair Bio Resources LLC) was purchased and thawed, pig skin from which fat was removed and extracted was prepared by removing the fat with scissors. The prepared microneedles were sealed and dried together with a desiccant for 3 days or longer and opened in an environment of a temperature of 25°C and a relative humidity of 60%, and then the pig skin from which fat was removed and extracted was punctured by the microneedles within 5 minutes after the microneedles were opened. At this time, the number of needles that were able to puncture the skin was counted and the results of evaluation performed based on the following standard were listed in Table 2.

A case where the skin was punctured by 80% or greater of all needles in the microneedle array: A
A case where the skin was punctured by 60% or greater and less than 80% of all needles in the microneedle array: B
A case where the skin was punctured by less than 60% of all needles in the microneedle array: C

Evaluation of solubility of human growth hormone (hGH)-containing microneedle array

The prepared microneedle array was dissolved in the above-described buffer solution and measured according to ELISA method. The recovery rate of the quantitative value of hGH actually measured in the microneedle array with respect to the theoretical value was used as an index of the solubility, and the results of the evaluation performed based on the following standard are listed in Table 2.

A case where the recovery rate was 95% or greater: A
A case where the recovery rate was 70% or greater and less than 95%: B
A case where the recovery rate was less than 70%: C

Blood kinetics test using microneedle array in mini pig

The effects of the example were evaluated by performing a blood kinetics test. A mini pig (Gottingen mini-pig, male, 5 weeks old, approximately 10 kg) was purchased, and a test of administering hGH was performed. The hair on the back of a mini pig was removed under anesthesia, the back was punctured by the microneedle array for 10 minutes. In order to evaluate relative bioavailability (BA) with respect to subcutaneous injection (S. C), the same amount of hGH as in target microneedles was subcutaneously injected to the mini pig under the same conditions as described above.
Blood sampling over time was performed by collecting 0.5 mL of blood at each time of 5 minutes, 15 minutes, 20 minutes, 30 minutes, 45 minutes, 1 hour, 2 hours, 4 hours, and 6 hours through a catheter before and after the administration. The plasma solution was recovered from the collected blood and the amount of hGH in the plasma was measured according to an ELISA method. The amount of hGH in the blood was graphed, the area under the blood concentration-time curve (AUC) was calculated, and then relative bioavailability with respect to subcutaneous injection was calculated. The results of evaluation performed based on the following standard are listed in the table 2.

A case where the relative bioavailability with respect to subcutaneous injection was 70% or greater: A

A case where the relative bioavailability with respect to subcutaneous injection was less than 70%: C

[Table 2]

| | Compositional ratio of needle tip portion | | | Mass ratio of water-soluble polymer in needle | Evaluation | | |
|---|---|---|---|---|---|---|---|
| | hGH | Water-soluble polymer | Disaccharides | | Puncture properties | Solubility | Drug efficacy |
| Comparative Example 1 | 1 | 1 | 0 | 45% | C | B | C |
| Comparative Example 2 | 1 | 1 | 0 | 70% | c | B | C |
| Comparative Example 3 | 1 | 0 | 0 | 70% | A | C | C |
| Comparative Example 4 | 1 | 0.5 | 0 | 45% | C | B | C |
| Comparative Example 5 | 1 | 2 | 2 | 60% | A | B | C |
| Example 1 | 1 | 0.9 | 0 | 70% | B | B | A |
| Example 2 | 1 | 0.05 | 0 | 70% | A | B | A |
| Example 3 | 1 | 0.5 | 0 | 55% | A | B | A |
| Example 4 | 1 | 0.5 | 0.25 | 85% | A | A | A |
| Example 5 | 1 | 0.5 | 4.5 | 75% | A | A | A |
| Example 6 | 1 | 0.5 | 5 | 75% | B | A | A |

Preparation of influenza vaccine (Flu)-containing microneedle array

(Production of mold)

A mold was prepared according to the same method as that for the human growth hormone (hGH)-containing microneedle array.

(Preparation of water-soluble polymer-dissolved solution containing influenza vaccine)

After seasonal three kinds of mixed influenza vaccines (Flu) (Denka Seiken Co., Ltd.) were concentrated by centrifugal ultrafiltration, an aqueous solution obtained by mixing hydroxyethyl starch (HES) (Fresenius Kabi) and sucrose (Suc) (Japanese Pharmacopoeia grade, Wako Pure Chemical Industries, Ltd.) was prepared. Each solution is as listed in the following table.

[Table 3]

|  | Flu | HES | Suc |
|---|---|---|---|
| Comparative Example 6 | 6.66 mg/mL | 6.66 mg/mL | 0.00 mg/mL |
| Comparative Example 7 | 6.66 mg/mL | 6.66 mg/mL | 0.00 mg/mL |
| Comparative Example 8 | 6.66 mg/mL | 0.00 mg/mL | 0.00 mg/mL |
| Comparative Example 9 | 6.66 mg/mL | 3.33 mg/mL | 0.00 mg/mL |
| Example 7 | 6.66 mg/mL | 5.99 mg/mL | 0.00 mg/mL |
| Example 8 | 6.66 mg/mL | 0.33 mg/mL | 0.00 mg/mL |
| Example 9 | 6.66 mg/mL | 3.33 mg/mL | 0.00 mg/mL |
| Example 10 | 6.66 mg/mL | 3.33 mg/mL | 3.33 mg/mL |
| Example 11 | 6.66 mg/mL | 3.33 mg/mL | 29.97 mg/mL |
| Example 12 | 6.66 mg/mL | 3.33 mg/mL | 33.30 mg/mL |

(Preparation of water-soluble polymer-dissolved solution forming sheet portion)

Chondroitin sulfate (manufactured by Maruha Nichiro Corporation) and disaccharides (sucrose) were dissolved in water at an arbitrary concentration ratio so as to have the composition listed in Table 4 at the time of formation of a microneedle array to prepare a water-soluble polymer-dissolved solution forming a sheet portion

(Filling and drying of polymer-dissolved solution containing medicament)

The procedures were carried out according to the same method as that for the human growth hormone (hGH)-containing microneedle array.

(Forming and drying of sheet portion)

The procedures were carried out according to the same method as that for the human growth hormone (hGH)-containing microneedle array.

(Peeling)

The procedures were carried out according to the same method as that for the human growth hormone (hGH)-containing microneedle array.

Distribution of needles of influenza vaccine (Flu)-containing microneedle array

The evaluation was performed according to the same method as that for the human growth hormone (hGH)-containing microneedle array.

Evaluation of puncture properties of influenza vaccine (Flu)-containing microneedle array

The evaluation was performed according to the same method as that for the human growth hormone (hGH)-containing microneedle array.

Evaluation of solubility of influenza vaccine (Flu)-containing microneedle array

The evaluation was performed according to the same method as that for the human growth hormone (hGH)-containing microneedle array.

Test of administering vaccine using influenza vaccine (Flu)-containing microneedle array

A mouse (Balb/c (CLEA Japan, Inc.), female, 7 weeks old) was purchased and a test for administering influenza vaccine to the mouse was performed after acclimation for 1 week. The hair on the back of the mouse was removed under anesthesia, the back was punctured by the microneedle array in a state in which the skin was stretched. In order to evaluate the amount of antibody to be produced with respect to subcutaneous injection (S. C), the same amount of vaccine as in target microneedles was subcutaneously injected to the mouse under the same conditions as described above.

Evaluation of amount of antibody to be produced in serum of mouse

After the administration, the mouse raised for 4 weeks was undergone laparotomy and the blood was collected from the posterior vena cava. The blood which was allowed to stand at room temperature was centrifuged, and the supernatant was collected to obtain the serum. The amount of antibody (IgG: immunoglobulin G) specific to the influenza contained in the obtained serum was measured according to the following technique.

(Measurement of amount of IgG antibody to be produced)

50 $\mu$L/well of the administered three kinds of mixed vaccines were dispensed to NUNC immunoplate maxisorp (C bottom, 96 well) and allowed to stand at 4°C overnight with a seal. After the plate was allowed to stand, the plate was washed with PBST (Gibco phosphate buffered saline (PBS) + 0.1% Tween (registered trademark)) four times. Thereafter, 200 $\mu$L/well of a blocking solution (50 mM trishydroxymethyl aminomethane (Tris) (pH of 8.0), 1% bovine serum albumin (BSA)) was added dropwise to the plate, and the plate was allowed to stand at room temperature for 30 minutes. After the plate was allowed to stand, the plate was washed with PBST (Gibco PBS + 0.1% Tween (registered trademark)) four times. After the washing, 100 $\mu$L/well of a sample obtained by being diluted approximately 10000 times with a diluent (50 mM Tris (pH of 8.0), 1% BSA, 0.1% Tween) was added dropwise and the plate was allowed to stand at room temperature for 1 hour. After the plate was allowed to stand, the plate was washed with PBST (Gibco PBS + 0.1% Tween) four times.

Next, 100 $\mu$L/well of horseradish peroxidase (HRP)-labeled anti-mouse IgG antibody (500 times) which was diluted with a diluent (50 mM Tris (pH of 8.0), 1% BSA, 0.1% Tween) was added dropwise, and the plate was allowed to stand at room temperature for 1 hour. After the plate was allowed to stand, the plate was washed with PBST (Gibco PBS + 0.1% Tween) four times in the same manner as described above.

Further, as a substrate reaction, 100 $\mu$L/well of 3,3',5,5'-tetramethylbenzidine (TMB) was added dropwise and the plate was allowed to stand at room temperature for 15 minutes under light shielding conditions. Next, 100 $\mu$L/well of a stop solution (1 mol/L HCl) was added dropwise.

After the dropwise addition, the absorbance (reference of 620 nm) at $\lambda$ of 450 nm was measured, and the amount of IgG antibody to be produced was relatively evaluated using the magnitude of absorbance based on the following standard. The results thereof are listed in Table 4.

The ratio of the amount of IgG antibody to be produced to the amount of IgG antibody to be produced in a case of subcutaneous injection was 90% or greater: A

The ratio of the amount of IgG antibody to be produced to the amount of IgG antibody to be produced in a case of subcutaneous injection was 70% or greater and less than 90%: B

The ratio of the amount of IgG antibody to be produced to the amount of IgG antibody to be produced in a case of subcutaneous injection was less than 70%: C

[Table 4]

| | Compositional ratio of needle tip portion | | | Mass ratio of water-soluble polymer in needle | Evaluation | | |
|---|---|---|---|---|---|---|---|
| | Flu | Water-soluble polymer | Disaccharides | | Puncture properties | Solubility | Drug efficacy |
| Comparative Example 6 | 1 | 1 | 0 | 45% | C | B | C |
| Comparative Example 7 | 1 | 1 | 0 | 80% | C | B | C |
| Comparative Example 8 | 1 | 0 | 0 | 80% | A | C | C |
| Comparative Example 9 | 1 | 0.5 | 0 | 45% | C | B | C |
| Example 7 | 1 | 0.9 | 0 | 80% | B | B | B |
| Example 8 | 1 | 0.05 | 0 | 80% | A | B | B |
| Example 9 | 1 | 0.5 | 0 | 55% | A | B | B |
| Example 10 | 1 | 0.5 | 0.5 | 80% | A | A | A |
| Example 11 | 1 | 0.5 | 4.5 | 80% | A | A | A |
| Example 12 | 1 | 0.5 | 5 | 80% | B | A | B |

From the results described above, it was understood that the drug efficacy was excellent in a case of the composition of each microneedle array of the examples compared to each group of the comparative examples. Particularly in a case where disaccharides were added as in Examples 4, 5, 10, and 11, the solubility of needles was unexpectedly improved and the medicament was able to be released from the needles of the microneedle array more effectively. However, in a case where the ratio of disaccharides to be added is extremely large as in Examples 6 and 12, the influence of moisture absorption due to humidity increases and the tip portion of each needle gradually becomes soft. Therefore, the puncture properties are slightly degraded. Further, sucrose has been exemplified as disaccharides in the examples, but the same effects at the time of using sucrose can be expected from maltose and trehalose. In Comparative Examples 1, 4, 6, and 9, since the amount of the water-soluble polymer in a needle was small, the strength of the entire needle was not able to be maintained so that the needle became soft. Further, in Comparative Examples 2 and 7, since the proportion of the water-soluble polymer in the tip portion was close to the concentration of the medicament, phase separation occurred and the tip portion became brittle. In Comparative Examples 3 and 8, since the base material did not contain a water-soluble polymer, the medicament was extremely condensed so that the solubility was significantly degraded. Further, in a case where the amount of the water-soluble polymer in the needle tip portion was excessive as in Comparative Example 5, a sufficient amount of medicament for exhibition of the drug efficacy was not able to be concentrated only on the needle tip portion, and thus the amount of medicament to be transferred to the blood at the time of administration was decreased. As described above, in order to obtain sufficient drug efficacy, the microneedle array having the composition of the present application, in which the entire needle and the needle tip portion are flexible and each needle is sufficiently dissolved in the skin after the skin is punctured so that the medicament can be released, becomes optimum.

Explanation of References

1:      microneedle array
2:      microneedle array
110:    microneedle
112:    needle
113:    frustum

116:     sheet portion
120:     layer containing medicament
122:     layer which does not contain medicament
W:       diameter (width)
H:       height
T:       height (thickness)
11:      original plate
12:      shaped portion
13:      mold
15:      needle-like recess
D:       diameter (diameter)
18:      mold complex
19:      air permeating sheet
20:      base
22:      water-soluble polymer-dissolved solution containing medicament
24:      water-soluble polymer-dissolved solution
29:      support
30:      tank
32:      pipe
34:      nozzle
34A:     lip portion
34B:     opening portion
36:      liquid supply device
P1:      pressing force
P2:      pressing pressure
P3:      pressing pressure
40:      base material
50:      truncated cone
52:      cone
D1:      diameter
D2:      diameter
L1:      pitch
HI:      height
H2:      height
61:      X-axis drive unit
62:      Z-axis drive unit
63:      nozzle
64:      liquid supply device
65:      suction stand
66:      laser displacement meter
67:      load cell
68:      control mechanism
69:      mold

**Claims**

1. A microneedle array comprising:

   a sheet portion; and
   at least one needle present on an upper surface of the sheet portion,
   wherein the needle contains a water-soluble polymer and a medicament,
   the water-soluble polymer occupies 50% by mass or greater of the total solid content of the needle,
   the water-soluble polymer and the medicament are present at a mass ratio of Formula 1, in a needle tip region which includes a needle tip of each needle and has a height corresponding to 20% of the total height of each needle, and
   the sheet portion contains a water-soluble polymer.
   Formula 1; water-soluble polymer:medicament = 0.05 to 0.9:1

**2.** The microneedle array according to claim 1,
wherein the needle contains a water-soluble polymer, disaccharides, and a medicament, and
the water-soluble polymer, the disaccharides, and the medicament are present at a mass ratio of Formula 2, in the needle tip region which includes a needle tip of each needle and has a height corresponding to 20% of the total height of each needle.
Formula 2; water-soluble polymer + disaccharides:medicament = 0.05 to 5:1

**3.** The microneedle array according to claim 1 or 2,
wherein the disaccharides is at least one selected from the group consisting of sucrose, maltose, and trehalose.

**4.** The microneedle array according to any one of claims 1 to 3,
wherein the water-soluble polymer in the needle is at least one selected from the group consisting of hydroxyethyl starch, hydroxypropyl methyl starch, pullulan, dextran, sodium chondroitin sulfate, sodium hyaluronate, carboxymethyl cellulose, polyvinylpyrrolidone, polyoxyethylene polyoxypropylene glycol, polyethylene glycol, and polyvinyl alcohol.

**5.** The microneedle array according to any one of claims 1 to 4,
wherein the water-soluble polymer in the sheet portion is at least one selected from the group consisting of hydroxyethyl starch, hydroxypropyl methyl starch, pullulan, dextran, sodium chondroitin sulfate, sodium hyaluronate, carboxymethyl cellulose, polyvinylpyrrolidone, polyoxyethylene polyoxypropylene glycol, polyethylene glycol, and polyvinyl alcohol.

**6.** The microneedle array according to any one of claims 1 to 5,
wherein the medicament is at least one selected from the group consisting of peptide hormones, vaccines, and adjuvants.

**7.** The microneedle array according to any one of claims 1 to 6,
wherein the needle contains two or more water-soluble polymers, disaccharides, and a medicament.

**8.** A method of producing the microneedle array comprising a sheet portion and at least one needle present on an upper surface of the sheet portion,
wherein the needle contains a water-soluble polymer and a medicament,
the water-soluble polymer occupies 50% by mass or greater of the total solid content of the needle,
the water-soluble polymer and the medicament are present at a mass ratio of Formula 1, in a needle tip region which includes a needle tip of each needle and has a height corresponding to 20% of the total height of each needle, and
the sheet portion contains a water-soluble polymer.

$$\text{Formula 1; water-soluble polymer:medicament} = 0.05 \text{ to } 0.9{:}1,$$

the method comprising: a step of forming some needles by drying a mold for forming needles, filled with a first water-soluble polymer-dissolved solution containing a medicament; and a step of filling the upper surface of some needles formed as described above with a second water-soluble polymer-dissolved solution and drying the surface.

**9.** The method according to claim 8,
wherein the needle contains a water-soluble polymer, disaccharides, and a medicament, and
the water-soluble polymer, the disaccharides, and the medicament are present at a mass ratio of Formula 2, in the needle tip region which includes a needle tip of each needle and has a height corresponding to 20% of the total height of each needle.

$$\text{Formula 2; water-soluble polymer} + \text{disaccharides:medicament} = 0.05 \text{ to } 5{:}1$$

**10.** The method according to claim 8 or 9, in which the mold for forming needles, filled with the first water-soluble polymer-dissolved solution containing a medicament is dried under a condition in which the moisture content of the first water-soluble polymer-dissolved solution reaches 20% or less after 30 to 300 minutes from the start of the drying.

**11.** The method according to any one of claims 8 to 10, in which the mold for forming needles, filled with the first water-soluble polymer-dissolved solution containing a medicament, is dried in a state in which the mold is covered by a container or accommodated in a container.

12. The method according to any one of claims 8 to 11, in which the mold for forming needles, filled with the first water-soluble polymer-dissolved solution containing a medicament is dried under a temperature condition of 1°C to 40°C at a relative humidity of 30% to 95%.

13. The method according to any one of claims 9 to 12, wherein the disaccharides is at least one selected from the group consisting of sucrose, maltose, and trehalose.

14. The method according to any one of claims 8 to 13,
wherein the water-soluble polymer in the needle is at least one selected from the group consisting of hydroxyethyl starch, hydroxypropyl methyl starch, pullulan, dextran, sodium chondroitin sulfate, sodium hyaluronate, carboxymethyl cellulose, polyvinylpyrrolidone, polyoxyethylene polyoxypropylene glycol, polyethylene glycol, and polyvinyl alcohol.

15. The method according to any one of claims 8 to 14,
wherein the water-soluble polymer in the sheet portion is at least one selected from the group consisting of hydroxyethyl starch, hydroxypropyl methyl starch, pullulan, dextran, sodium chondroitin sulfate, sodium hyaluronate, carboxymethyl cellulose, polyvinylpyrrolidone, polyoxyethylene polyoxypropylene glycol, polyethylene glycol, and polyvinyl alcohol.

## Patentansprüche

1. Mikronadelanordnung, enthaltend:

einen Lagenbereich und
zumindest eine Nadel, die auf einer oberen Oberfläche des Lagenbereiches vorhanden ist,
worin die Nadel ein wasserlösliches Polymer und ein Medikament enthält,
das wasserlösliche Polymer 50 Masse-% oder mehr des gesamten Feststoffgehaltes der Nadel besetzt,
das wasserlösliche Polymer und das Medikament bei einem Massenverhältnis der Formel 1 in einem Nadelspitzenbereich vorhanden sind, der eine Nadelspitze einer jeden Nadel enthält und eine Höhe hat, die 20 % der Gesamthöhe einer jeden Nadel entspricht, und
der Lagenbereich ein wasserlösliches Polymer enthält,

```
Formel 1: wasserlösliches Polymer:Medikament = 0,05 bis
0,9:1.
```

2. Mikronadelanordnung gemäß Anspruch 1, worin die Nadel ein wasserlösliches Polymer, Disaccharide und ein Medikament enthält, und
das wasserlösliche Polymer, die Disaccharide und das Medikament bei einem Massenverhältnis gemäß Formel 2 in dem Nadelspitzenbereich vorhanden sind, der eine Nadelspitze einer jeden Nadel enthält und eine Höhe hat, die 20 % der Gesamthöhe einer jeden Nadel entspricht:

```
Formel 2: wasserlösliches Polymer +
Disaccharide:Medikament = 0,05 bis 5:1.
```

3. Mikronadelanordnung gemäß Anspruch 1 oder 2, worin die Disaccharide zumindest eines sind, ausgewählt aus der Gruppe, bestehend aus Sucrose, Maltose und Trehalose.

4. Mikronadelanordnung gemäß einem der Ansprüche 1 bis 3,
worin das wasserlösliche Polymer in der Nadel zumindest eines ist, ausgewählt aus der Gruppe, bestehend aus Hydroxyethylstärke, Hydroxypropylmethylstärke, Pullulan, Dextran, Natriumchondroitinsulfat, Natriumhyaluronat, Carboxymethylcellulose, Polyvinylpyrrolidon, Polyoxyethylenpolyoxypropylenglykol, Polyethylenglykol und Polyvinylalkohol.

**5.** Mikronadelanordnung gemäß einem der Ansprüche 1 bis 4,
worin das wasserlösliche Polymer im Lagenbereich zumindest eines ist, ausgewählt aus der Gruppe, bestehend aus Hydroxyethylstärke, Hydroxypropylmethylstärke, Pullulan, Dextran, Natriumchondroitinsulfat, Natriumhyaluronat, Carboxymethylcellulose, Polyvinylpyrrolidon, Polyoxyethylenpolyoxypropylenglykol, Polyethylenglykol und Polyvinylalkohol.

**6.** Mikronadelanordnung gemäß einem der Ansprüche 1 bis 5, worin das Medikament zumindest eines ist, ausgewählt aus der Gruppe, bestehend aus Peptidhormonen, Vakzinen und Adjuvantien.

**7.** Mikronadelanordnung gemäß einem der Ansprüche 1 bis 6, worin die Nadel zwei oder mehrere wasserlösliche Polymer, Disaccharide und ein Medikament enthält.

**8.** Verfahren zur Erzeugung der Mikronadelanordnung, enthaltend einen Lagenbereich und zumindest eine Nadel, die auf einer oberen Oberfläche des Lagenbereiches vorhanden ist,
worin die Nadel ein wasserlösliches Polymer und ein Medikament enthält,
das wasserlösliche Polymer 50 Masse-% oder mehr des gesamten Feststoffgehaltes der Nadel besetzt,
das wasserlösliche Polymer und das Medikament bei einem Massenverhältnis der Formel 1 in einem Nadelspitzenbereich vorhanden sind, der eine Nadelspitze einer jeden Nadel enthält und eine Höhe hat, die 20 % der Gesamthöhe einer jeden Nadel entspricht, und
das Lagenbereich ein wasserlösliches Polymer enthält,

```
Formel 1: wasserlösliches Polymer:Medikament = 0,05 bis
0,9:1
```

wobei das Verfahren enthält: einen Schritt zur Bildung von einigen Nadeln durch Trocknen einer Form zur Bildung von Nadeln, gefüllt mit einer ersten wasserlöslichen Polymer-aufgelösten Lösung, die ein Medikament enthält, und einen Schritt zum Füllen der oberen Oberfläche von einigen Nadeln, die wie oben beschrieben gebildet sind, mit einer zweiten wasserlöslichen Polymer-aufgelösten Lösung und Trocknen der Oberfläche.

**9.** Verfahren gemäß Anspruch 8, worin die Nadel ein wasserlösliches Polymer, Disaccharide und ein Medikament enthält, und
das wasserlösliche Polymer, die Disaccharide und das Medikament bei einem Massenverhältnis gemäß Formel 2 in dem Nadelspitzenbereich vorhanden sind, der eine Nadelspitze einer jeden Nadel enthält und eine Höhe hat, die 20 % der Gesamthöhe einer jeden Nadel entspricht:

```
Formel 2: wasserlösliches Polymer +
Disaccharide:Medikament = 0,05 bis 5:1
```

**10.** Verfahren gemäß Anspruch 8 oder 9, worin die Form zur Bildung von Nadeln, gefüllt mit der ersten wasserlöslichen Polymer-aufgelösten Lösung, die ein Medikament enthält, unter einer Bedingung getrocknet wird, worin der Feuchtigkeitsgehalt der ersten wasserlöslichen Polymer-aufgelösten Lösung 20 % oder weniger 30 bis 300 Minuten nach Beginn des Trocknens erreicht.

**11.** Verfahren gemäß einem der Ansprüche 8 bis 10, worin die Form zur Bildung von Nadeln, gefüllt mit der ersten wasserlöslichen Polymer-aufgelösten Lösung, die ein Medikament enthält, in einem Zustand getrocknet wird, bei dem die Form durch einen Behälter bedeckt oder in einem Behälter enthalten ist.

**12.** Verfahren gemäß einem der Ansprüche 8 bis 11, worin die Form zur Bildung von Nadeln, gefüllt mit der ersten wasserlöslichen Polymer-aufgelösten Lösung, die ein Medikament enthält, bei einer Temperaturbedingung von 1 bis 40°C bei einer relativen Feuchtigkeit von 30 bis 95 % getrocknet wird.

**13.** Verfahren gemäß einem der Ansprüche 9 bis 12, worin die Disaccharide zumindest eines sind, ausgewählt aus der Gruppe, bestehend aus Sucrose, Maltose und Trehalose.

**14.** Verfahren gemäß einem der Ansprüche 8 bis 13,
worin das wasserlösliche Polymer in der Nadel zumindest eines ist, ausgewählt aus der Gruppe, bestehend aus Hydroxyethylstärke, Hydroxypropylmethylstärke, Pullulan, Dextran, Natriumchondroitinsulfat, Natriumhyaluronat, Carboxymethylcellulose, Polyvinylpyrrolidon, Polyoxyethylenpolyoxypropylenglykol, Polyethylenglykol und Polyvinylalkohol.

15. Verfahren gemäß einem der Ansprüche 8 bis 14,
worin das wasserlösliche Polymer im Lagenbereich zumindest eines ist, ausgewählt aus der Gruppe, bestehend aus Hydroxyethylstärke, Hydroxypropylmethylstärke, Pullulan, Dextran, Natriumchondroitinsulfat, Natriumhyaluronat, Carboxymethylcellulose, Polyvinylpyrrolidon, Polyoxyethylenpolyoxypropylenglykol, Polyethylenglykol und Polyvinylalkohol.

**Revendications**

1. Réseau de micro-aiguilles comprenant :

   une partie de feuille ; et
   au moins une aiguille présente sur une surface supérieure de la partie de feuille,
   dans lequel l'aiguille contient un polymère hydrosoluble et un médicament,
   le polymère hydrosoluble occupe 50 % en masse ou plus de la teneur totale en matières solides de l'aiguille,
   le polymère hydrosoluble et le médicament sont présents à un rapport de masse de la formule 1, dans une région de pointe d'aiguille qui inclut une pointe d'aiguille de chaque aiguille et présente une hauteur correspondant à 20 % de la hauteur totale de chaque aiguille, et
   la partie de feuille contient un polymère hydrosoluble.

   Formule 1 ; polymère hydrosoluble:médicament = entre 0,05 et 0,9:1.

2. Réseau de micro-aiguilles selon la revendication 1,
   dans lequel l'aiguille contient un polymère hydrosoluble, des disaccharides et un médicament, et
   le polymère hydrosoluble, les disaccharides et le médicament sont présents à un rapport de masse de la formule 2, dans la région de pointe d'aiguille qui inclut une pointe d'aiguille de chaque aiguille et présente une hauteur correspondant à 20 % de la hauteur totale de chaque aiguille.

   Formule 2 ; polymère hydrosoluble + disaccharides:médicament = entre 0,05 et 5:1

3. Réseau de micro-aiguilles selon la revendication 1 ou 2,
   dans lequel les disaccharides sont au moins un élément sélectionné dans le groupe constitué par le sucrose, le maltose et le tréhalose.

4. Réseau de micro-aiguilles selon l'une quelconque des revendications 1 à 3,
   dans lequel le polymère hydrosoluble dans l'aiguille est au moins un élément sélectionné dans le groupe constitué par l'amidon d'hydroxyéthyle, l'amidon d'hydroxypropyle méthyle, le pullulane, la dextrane, le sulfate de chondroïtine de sodium, l'hyaluronate de sodium, la carboxyméthylecellulose, la polyvinylpyrrolidone, le polyoxyéthylène polyoxypropylène glycol, le polyéthylène glycol et l'alcool polyvinylique.

5. Réseau de micro-aiguilles selon l'une quelconque des revendications 1 à 4,
   dans lequel le polymère hydrosoluble dans la partie de feuille est au moins un élément sélectionné dans le groupe constitué par l'amidon d'hydroxyéthyle, l'amidon d'hydroxypropyle méthyle, le pullulane, la dextrane, le sulfate de chondroïtine de sodium, l'hyaluronate de sodium, la carboxyméthylecellulose, la polyvinylpyrrolidone, le polyoxyéthylène polyoxypropylène glycol, le polyéthylène glycol et l'alcool polyvinylique.

6. Réseau de micro-aiguilles selon l'une quelconque des revendications 1 à 5,
   dans lequel le médicament est au moins un élément sélectionné dans le groupe constitué par des hormones peptidiques, des vaccins et des adjuvants.

7. Réseau de micro-aiguilles selon l'une quelconque des revendications 1 à 6,
   dans lequel l'aiguille contient au moins deux polymères hydrosolubles, des disaccharides et un médicament.

8. Procédé de production du réseau de micro-aiguilles comprenant une partie de feuille et au moins une aiguille présente sur une surface supérieure de la partie de feuille,
dans lequel l'aiguille contient un polymère hydrosoluble et un médicament,
le polymère hydrosoluble occupe 50 % en masse ou plus de la teneur totale en matières solides de l'aiguille,
le polymère hydrosoluble et le médicament sont présents à un rapport de masse de la formule 1, dans une région de pointe d'aiguille qui inclut une pointe d'aiguille de chaque aiguille et présente une hauteur correspondant à 20 % de la hauteur totale de chaque aiguille, et
la partie de feuille contient un polymère hydrosoluble.

Formule 1 ; polymère hydrosoluble:médicament = entre 0,05 et 0,9:1,

le procédé comprenant : une étape consistant à former quelques aiguilles en séchant un moule pour former des aiguilles, remplies d'une première solution dissoute dans un polymère hydrosoluble contenant un médicament; et une étape consistant à remplir la surface supérieure de certaines aiguilles formées telles que décrites ci-dessus avec une seconde solution dissoute dans un polymère hydrosoluble et à sécher la surface.

9. Procédé selon la revendication 8,
dans lequel l'aiguille contient un polymère hydrosoluble, des disaccharides et un médicament, et
le polymère hydrosoluble, les disaccharides et le médicament sont présents à un rapport de masse de la formule 2, dans la région de pointe d'aiguille qui inclut une pointe d'aiguille de chaque aiguille et présente une hauteur correspondant à 20 % de la hauteur totale de chaque aiguille.

Formule 2 ; polymère hydrosoluble + disaccharides:médicament = entre 0,05 et 5:1.

10. Procédé selon la revendication 8 ou 9, dans lequel le moule pour former des aiguilles, remplies de la première solution dissoute dans un polymère hydrosoluble contenant un médicament, est séché dans une condition dans laquelle la teneur en humidité de la première solution dissoute dans un polymère hydrosoluble atteint 20 % ou moins après 30 à 300 minutes depuis le commencement du séchage.

11. Procédé selon l'une quelconque des revendications 8 à 10, dans lequel le moule pour former des aiguilles, remplies de la première solution dissoute dans un polymère hydrosoluble contenant un médicament, est séché dans un état dans lequel le moule est recouvert par un récipient ou est logé dans un récipient.

12. Procédé selon l'une quelconque des revendications 8 à 11, dans lequel le moule pour former des aiguilles, remplies de la première solution dissoute dans un polymère hydrosoluble contenant un médicament, est séché à une condition de température comprise entre 1 °C et 40 °C à une humidité relative comprise entre 30 % et 95 %.

13. Procédé selon l'une quelconque des revendications 9 à 12, dans lequel les disaccharides sont au moins un élément sélectionné dans le groupe constitué par le sucrose, le maltose et le tréhalose.

14. Procédé selon l'une quelconque des revendications 8 à 13,
dans lequel le polymère hydrosoluble dans l'aiguille est au moins un élément sélectionné dans le groupe constitué par l'amidon d'hydroxyéthyle, l'amidon d'hydroxypropyle méthyle, le pullulane, la dextrane, le sulfate de chondroïtine de sodium, l'hyaluronate de sodium, la carboxyméthylecellulose, la polyvinylpyrrolidone, le polyoxyéthylène polyoxypropylène glycol, le polyéthylène glycol et l'alcool polyvinylique.

15. Procédé selon l'une quelconque des revendications 8 à 14,
dans lequel le polymère hydrosoluble dans la partie de feuille est au moins un élément sélectionné dans le groupe constitué par l'amidon d'hydroxyéthyle, l'amidon d'hydroxypropyle méthyle, le pullulane, la dextrane, le sulfate de chondroïtine de sodium, l'hyaluronate de sodium, la carboxyméthylecellulose, la polyvinylpyrrolidone, le polyoxyéthylène polyoxypropylène glycol, le polyéthylène glycol et l'alcool polyvinylique.

## FIG. 1

## FIG. 2A

## FIG. 2B

## FIG. 2C

## FIG. 3

## FIG. 4

## FIG. 5

# FIG. 6

# FIG. 7

# FIG. 8

EP 3 348 256 B1

## FIG. 9

## FIG. 10A

## FIG. 10B

## FIG. 10C

30

## FIG. 11

## FIG. 12

(A)

(B)

## FIG. 13A

## FIG. 13B

## FIG. 13C

## FIG. 14

## FIG. 15

FIG. 16

## FIG. 17A

34

22

15

13

## FIG. 17B

120

13

## FIG. 17C

24

120

13

## FIG. 17D

1

116 { 122

113 {

112 {

120

13

# FIG. 18A

# FIG. 18B

# FIG. 18C

# FIG. 19

## FIG. 20

## FIG. 21

## FIG. 22A

## FIG. 22B

# FIG. 23

**REFERENCES CITED IN THE DESCRIPTION**

**Patent documents cited in the description**

- JP 2011224332 A
- JP 2005154321 A
- JP 5587647 B
- JP 2013162982 A
- JP 2013153866 A
- WO 2014077242 A